# EUROPEAN PATENT APPLICATION

(11) **EP 4 699 516 A1**
(43) Date of publication of application: **25.02.2026**
(21) Application number: 24796067.7
(22) Date of filing: 23.04.2024
(51) Int. Cl.: A61B 1/015, A61B 1/018, A61B 1/00, A61B 1/04, A61B 1/06, A61B 1/303

(54) **VIDICON AND ENDOSCOPE**

(30) Priority: 23.04.2023 CN 202310443161; 23.04.2023 CN 202310443170; 23.04.2023 CN 202310443181; 23.04.2023 CN 202310443189
(71) Applicant: Changsha Magill Medical Technology Co., Ltd., Changsha, Hunan 410205 (CN)
(72) Inventor: LI, Daqing, Changsha, Hunan 410205 (CN); XU, Xiong, Changsha, Hunan 410205 (CN); LIU, Zhonghui, Changsha, Hunan 410205 (CN)
(74) Representative: Gevers Patents
(86) International application number: PCT/CN2024/089364
(87) International publication number: WO 2024/222687

(57) **Abstract**

Provided in the present application are a vidicon and an endoscope. The vidicon is configured to be in insertion-pull fit with an endoscope mounting assembly, and comprises a core tube and an elastic member, wherein the core tube comprises a main insertion section and a head end section in the lengthwise direction of the core tube; the head end section is connected to a distal end of the main insertion section in a sleeved manner; the head end section and/or the main insertion section can axially move close to each other under the action of an external force; and the elastic member is arranged in the core tube, and can be compressed and reset so as to drive the head end section to tend to axially move away from the main insertion section. The vidicon provided in the embodiments of the present application has a simple structure, and is adaptive in terms of length, such that accurate and appropriate fitting between the tail end of the vidicon and a lumen, in which the vidicon is mounted, of the endoscope mounting assembly is realized; and the problem of the adaptability being reduced due to manufacturing and assembly errors can be effectively solved, thereby being convenient for the vidicon to acquire images with high quality, and reducing the probability of damage to the vidicon caused by a hard bump and collision when the vidicon is mounted.

## Description

### CROSS-REFERENCE TO RELATED APPLICATIONS

This application claims priority to Chinese Patent Application No. 202310443161.7, Chinese Patent Application No. 202310443181.4, Chinese Patent Application No. 202310443170.6 and Chinese Patent Application No. 202310443189.0, filed on April 23, 2023, the contents of which are hereby incorporated by reference in their entirety.

### TECHNICAL FIELD

The present application relates to the technical field of medical devices, and in particular to an image tube and an endoscope.

### BACKGROUND

In the related art, an endoscope generally includes an insertion tube that extends into a human body or an animal body through a natural lumen or a surgical stoma of the human body or the animal body. The endoscope is provided with a working channel tube, a liquid inlet channel and a visual device. The endoscope is usually used as reusable equipment. After the endoscope is used, a high-level disinfection and sterilization measure, such as immersion disinfection, generally need to be adopted. However, a narrow and complex channel structure in the endoscope has a risk of cross-infection caused by incomplete disinfection and sterilization. The high-level disinfection and sterilization measure have a cumbersome procedure and a high cost. In the related art, the entire endoscope is used as a disposable consumable, and is discarded after the entire endoscope is used. In this way, although the problem of high-level disinfection and sterilization is avoided, the use cost is still very high.

In order to reduce the problem of disinfection and sterilization, reduce the probability of cross-infection and reduce the use cost, a visual sleeve is disposed inside the insertion tube of the endoscope, the visual sleeve is arranged to sheathe an image tube and is provided with a transparent optical window to close a distal end of the visual sleeve. A high-cost image tube and a related image system are designed as reusable instruments, and a low-cost visual sleeve and the insertion tube and the like are designed as disposable instruments, to reduce the probability of cross-infection and the use cost. However, the endoscope requires that an optical system of a tail end of the image tube is accurately and appropriately fitted against the optical window of the visual sleeve. If the optical system of the tail end of the image tube is not well fitted with the optical window of the visual sleeve, for example, the optical system of the tail end of the image tube is not fully fitted against the optical window of the visual sleeve, or there is an excessive force between the optical system of the tail end of the image tube and the optical window of the visual sleeve, the imaging of the image tube may be seriously affected, and even the optical system of the tail end of the image tube and the optical window of the visual sleeve may be damaged.

Information disclosed in the background is merely intended to increase understanding of the general background of the present application and should not be construed as an acknowledgement or in any way implication that the information has become the related art well known to those skilled in the art.

### SUMMARY

Embodiments of the present application desire to provide an image tube which is adaptive in terms of length, has a simple structure and a low cost, and an endoscope. In order to achieve the above objects, technical solutions of the embodiments of the present application are realized as follows.

An image tube configured to be in insertion-extraction fit with an endoscope fitting assembly includes a core tube and an elastic member.

The core tube includes a main insertion section and a head end section in a lengthwise direction of the core tube. A proximal end of the head end section is connected to a distal end of the main insertion section. The head end section and/or the main insertion section is/are axially movable close to each other under an action of an external force.

The elastic member is disposed in the core tube. The elastic member is compressible and resilient, to actuate the head end section producing moving tendency which causes the head end section to axially be away from the main insertion section.

In some embodiments, the head end section includes a first body tube and a first casing tube. The first casing tube is provided as a separate component and fixed to a proximal end of the first body tube.

The main insertion section includes a second body tube and a second casing tube. The second casing tube is provided as a separate component and fixed to a distal end of the second body tube. The head end section and the main insertion section are connected to each other through the first casing tube and the second casing tube.

In some embodiments, a first dwindling section is formed at the distal end of the second body tube. The first dwindling section is inserted inside a proximal end of the second casing tube, and the first casing tube is arranged inside a distal end of the second casing tube. One end of the elastic member is kept against a distal end portion of the second body tube, and another end of the elastic member is kept against a proximal end portion of the first casing tube.

In some embodiments, the second casing tube is provided with an accommodating lumen in which the first dwindling section is accommodated. The first casing tube includes a first casing section and a second casing section in a lengthwise direction of the first casing tube, and an outer diameter of the first casing section is greater than an outer diameter of the second casing section. The first casing section is inserted into the accommodating lumen, and a distal side wall of the accommodating lumen protrudes inwardly to form a limiting wall. A proximal end portion of the first casing section is kept against the elastic member, and a distal end portion of the first casing section detachably abuts against the limiting wall.

In some embodiments, a circumferential outer side wall of the first casing section is tangent to a side wall of the accommodating lumen.

In some embodiments, a second dwindling section is formed at a proximal end of the second casing tube. The second dwindling section is inserted inside the distal end of the second body tube, and the first casing tube is arranged around a distal end of the second casing tube. One end of the elastic member is kept against a distal end portion of the second body tube, and another end of the elastic member is kept against a proximal end portion of the first casing tube.

In some embodiments, the second casing tube is arranged around the first casing tube, a distal end portion of the second casing tube is axially spaced apart from a proximal end portion of the first body tube to define a movement stroke of the head end section in an axial direction; or the first casing tube is arranged around the second casing tube, and a proximal end portion of the first casing tube is axially spaced apart from a distal end portion of the second body tube to define a movement stroke of the head end section in an axial direction.

In some embodiments, the elastic member is a compression spring.

In some embodiments, a distal end of the head end section is integrated with an optical component, and a photoelectric conversion component for converting an optical signal of the optical component into an electrical signal. The image tube includes a signal line disposed in the core tube and extending along the lengthwise direction of the core tube. A distal end of the signal line is connected to the photoelectric conversion component.

In some embodiments, the image tube includes a disc part connected to a proximal end of the core tube. The disc part includes a disc body and a protrusion protruding in a direction from the disc body to the head end section. A distal end of the protrusion is provided with a plurality of first electrical terminals, and a proximal end of the signal line is electrically connected to the plurality of first electrical terminals.

The embodiments of the present application provides an endoscope, which includes:
an endoscope fitting assembly and the image tube according to any one of embodiments of the present application.

The endoscope fitting assembly includes:
a handle assembly, an insertion tube assembly and a visual sleeve.

A proximal end of the insertion tube assembly is connected to the handle assembly. The insertion tube assembly includes an insertion tube, and a distal end surface of the insertion tube is provided with a liquid outlet.

The visual sleeve is disposed inside the insertion tube, and the visual sleeve is provided with a visual channel. A distal end portion of the visual sleeve is provided with a closed optical window.

The core tube is insertable into the visual channel, and a distal end portion of the head end section is configured to be kept against an inner surface of the optical window.

In some embodiments, the visual sleeve is provided with a visual insertion-extraction port at a proximal end of the handle assembly. The image tube is in detachable insertion-extraction fit with the visual channel through the visual insertion-extraction port.

In some embodiments, the visual sleeve includes a first visual sleeve and a second visual sleeve connected to each other along a lengthwise direction of the visual sleeve. A proximal end of the first visual sleeve is provided with the visual insertion-extraction port at the proximal end of the handle assembly. The first visual sleeve is provided with a first visual channel, a proximal end of the second visual sleeve extends into and is fixed in the first visual channel, and another end of the second visual sleeve is disposed in the insertion tube. A cross-section of the first visual channel gradually decreases in a direction from a proximal end to a distal end.

In some embodiments, the visual sleeve is provided with a plurality of second electrical terminals. The image tube includes a disc part, and the disc part includes a disc body, and a protrusion protruding from the disc body toward the visual sleeve. A side of the protrusion facing toward the visual sleeve is integrated with a plurality of first electrical terminals, and each of the plurality of first electrical terminals is detachably connected to and conductively connected to a respective one of the plurality of second electrical terminals. The endoscope fitting assembly includes a data exchange interface configured to interact with an external device, and the data exchange interface is in electrical communication with the plurality of second electrical terminals.

In some embodiments, a proximal end of the visual sleeve extends out of a proximal end portion of the handle assembly. A proximal end portion of the visual sleeve is provided with a recess and the visual insertion-extraction port, and the proximal end portion of the visual sleeve is recessed toward a direction close to a distal end of the insertion tube to form the recess. The plurality of second electrical terminals are disposed at a distal end of the recess and penetrate through a groove wall of the recess along an insertion-extraction direction of the image tube. One end of each second electrical terminal is exposed in the recess, and another end of each second electrical terminal extends into a space in the handle assembly. The protrusion extends into and is fixed in the recess.

In some embodiments, the endoscope fitting assembly includes a cable, and a light emitter configured to provide illumination to a field of view of the image tube. The light emitter is disposed at a distal end of the insertion tube. One end of the cable is electrically connected to the light emitter, and another end of the cable extends to the handle assembly.

In some embodiments, the endoscope fitting assembly includes a data exchange interface configured to interact with an external device. The cable is electrically connected to the data exchange interface.

In some embodiments, the optical window includes an incident lens element, and a reflecting prism disposed in the visual sleeve. The incident lens element is disposed at an end of the visual sleeve and located on a light incident side of the reflecting prism. The incident lens element is configured to receive external light, and refract and converge the external light to the reflecting prism, and the reflecting prism is configured to transmit light to a light incident portion of the image tube in the visual channel by reflection.

In some embodiments, a light emitting surface of the reflecting prism is perpendicular to a lengthwise direction of the visual sleeve.

In some embodiments, a distal end surface of the incident lens element is an inclined surface, and an arc-shaped groove is formed at a side of the incident lens element facing toward the reflecting prism. A surface of the arc-shaped groove is a light emitting surface of the incident lens element, and a light incident surface of the reflecting prism covers the arc-shaped groove.

In some embodiments, the reflecting prism is provided with at least a first reflecting surface and a second reflecting surface disposed face to face. The first reflecting surface is inclined toward the arc-shaped groove, and the second reflecting surface is located on a light emitting side of the first reflecting surface, to allow light emitted from the arc-shaped groove to be emitted after being reflected by the first reflecting surface and the second reflecting surface in sequence, or to be emitted after being subjected to one or more light processing.

In some embodiments, the handle assembly is provided with a liquid inlet channel, and an interior of the insertion tube is provided with a free space in communication with the liquid inlet channel and the liquid outlet. The endoscope fitting assembly includes a working channel tube, and a working channel through which a surgical instrument passes is formed inside the working channel tube. The working channel tube is arranged inside the insertion tube and is provided with an outlet at the distal end surface of the insertion tube. The free space is an unoccupied space in the insertion tube.

In some embodiments, the endoscope fitting assembly includes a joint, and the handle assembly is provided with a mounting port. The joint is rotatably arranged in the mounting port, and a proximal end of the insertion tube is hermetically inserted into and fixed in the joint.

In some embodiments, the joint is provided with a flow channel into which an end of the insertion tube extends. The endoscope fitting assembly includes a liquid inlet tube, one end of the liquid inlet tube is configured to introduce an external fluid, and another end of the liquid inlet tube is connected to an inlet of the flow channel. The flow channel and a space in the liquid inlet tube define at least a portion of the liquid inlet channel.

In some embodiments, the handle assembly includes a handle, and a handgrip disposed at a bottom side of the handle. The working channel tube and the visual sleeve extend along a lengthwise direction of the handle. The mounting port is disposed at an end of the handle close to the insertion tube, at least a portion of the joint is disposed inside a distal end of the handle, and an end of the liquid inlet tube away from the joint extends along the handgrip to an end of the handgrip away from the handle.

In some embodiments, the endoscope fitting assembly includes a turntable disposed outside the handle assembly. The turntable is arranged to cover around an outer peripheral side of the insertion tube, and the turntable is rotatably arranged to case around the joint and detachably fixed to the joint.

In some embodiments, the endoscope fitting assembly includes an end cap. A proximal end surface of the handle assembly is provided with a first snapping part, the end cap is arranged to cover around the disc part, and a circumferential surface of the end cap is provided with a second snapping part. The first snapping part is detachably snapped to the second snapping part, to allow the disc part to be kept against a proximal end portion of the visual sleeve.

In some embodiments, the insertion tube assembly includes a fixing support disposed inside a distal end of the insertion tube. The fixing support is connected to an inner wall of the insertion tube, and the fixing support is provided with a first hole and a second hole. The working channel tube is constrained in the first hole, and the visual sleeve is constrained in the second hole, to allow a distal end of the working channel tube and a distal end of the visual sleeve to be fixed inside the insertion tube.

In some embodiments, a portion of an outer circumferential surface of the fixing support is recessed toward a direction away from the inner wall of the insertion tube to form a recessed region. The recessed region and the inner wall of the insertion tube opposite to the recessed region define the liquid outlet.

In some embodiments, the fixing support includes two mounting blocks separated from each other. The working channel tube and the visual sleeve are arranged along a first direction, and the two mounting blocks are disposed opposite to each other along a second direction and define the first hole and the second hole. The first direction is perpendicular to the second direction.

In some embodiments, an outer circumferential surface of each mounting block facing toward the inner wall of the insertion tube is provided with an arc-shaped surface. The arc-shaped surface is fitted against the inner wall of the insertion tube.

In some embodiments, the endoscope fitting assembly includes a cable, and a light emitter configured to provide illumination to the image tube. The fixing support is provided with a through hole penetrating through the fixing support along an axial direction of the insertion tube. The cable passes through the through hole and is connected to the light emitter. The inner wall of the insertion tube, an outer wall of the working channel tube, and an outer wall of the visual sleeve form a triangular region in which the cable is arranged.

In some embodiments, the light emitter includes a circuit board, and a light emitting chip integrated on the circuit board. The circuit board is attached to a distal end surface of the fixing support, and a surface of the circuit board and a surface of the light emitting chip are coated with a glue filling layer.

In some embodiments, the insertion tube assembly includes a glue filling layer. The glue filling layer is formed by filling glue into a region defined by a distal end surface of the fixing support, and the inner wall of the insertion tube and an outer wall of the optical window. An outer surface of the glue filling layer forms at least a portion of the distal end surface of the insertion tube.

In some embodiments, the visual sleeve includes a sheath and the optical window. A distal end of the sheath is open, and the optical window closes a distal end portion of the sheath. The glue filling layer is arranged between a circumference of the optical window and the inner wall of the insertion tube.

In some embodiments, the endoscope fitting assembly includes a coupling part fixed to the handle assembly and a liquid discharging tube. The coupling part includes a first coupling sleeve and a second coupling sleeve butted with each other. A distal end of the first coupling sleeve is provided with an insertion cavity, and a proximal end of the second coupling sleeve is inserted into the insertion cavity. A cavity region is formed between a portion of a circumferential surface of the second coupling sleeve and an inner wall of the insertion cavity. A side wall of the insertion cavity corresponding to the cavity region is provided with a coupling interface. A proximal end of the working channel tube extends into and is fixed inside a distal end of the second coupling sleeve, and is in communication with the cavity region. A space expanding section that expands toward the first coupling sleeve is formed inside the proximal end of the second coupling sleeve.

An end of the liquid discharging tube is in communication with the coupling interface, to discharge fluid from the working channel tube.

In some embodiments, a cross-sectional area of the space expanding section increases gradually in a direction away from the working channel tube.

In some embodiments, the second coupling sleeve includes a hermitic axial section, and a circumferential surface of the hermitic axial section is in hermitic interference fit with the insertion cavity. An axial end surface of the hermitic axial section is spaced apart from a cavity end surface of the insertion cavity facing toward the axial end surface, and the coupling interface is disposed at a bottom of a circumferential side wall between the axial end surface and the cavity end surface.

In some embodiments, the second coupling sleeve includes a dwindling axial section connected to a proximal end of the hermitic axial section, and an outer diameter of the dwindling axial section is less than an outer diameter of the hermitic axial section. The space expanding section extends from an interior of the hermitic axial section to a proximal end surface of the dwindling axial section.

In some embodiments, the first coupling sleeve is provided with a first axial hole, and the insertion cavity is positioned at a distal end of the first axial hole. An inner diameter of the insertion cavity is greater than an inner diameter of the distal end of the first axial hole, and the cavity end surface is formed at a boundary of the insertion cavity and the first axial hole. The first coupling sleeve is provided with an annular platform positioned in the insertion cavity, the annular platform surrounds a distal end portion of the first axial hole, and the annular platform is spaced apart from a circumferential side wall of the insertion cavity.

In some embodiments, an end surface of the annular platform is spaced apart from an end face of the dwindling axial section.

In some embodiments, a cross-sectional area of the first axial hole decreases continuously in a direction from a proximal end to the distal end of the first axial hole.

In some embodiments, the endoscope fitting assembly includes a screw cap detachably arranged on a proximal end of the first coupling sleeve. The screw cap includes a cap body and a bulge protruding from an interior of the cap body toward the first coupling sleeve. The bulge extends into the first axial hole and seals the first axial hole.

In some embodiments, the handle assembly includes a handle, and a handgrip disposed at a bottom side of the handle. The working channel tube and the visual sleeve extend along a lengthwise direction of the handle. An end of the liquid discharging tube away from the coupling part extends along the handgrip to an end of the handgrip away from the handle.

In some embodiments, the endoscope fitting assembly includes a coupling head. A first coupling cavity and a second coupling cavity in communication with each other are formed inside the coupling head. The first coupling cavity is in communication with the coupling interface, and the second coupling cavity is in communication with an end of the liquid discharging tube close to the handle. The first coupling cavity is disposed along a lengthwise direction of the handgrip, and the second coupling cavity is disposed along the lengthwise direction of the handle.

In some embodiments, the liquid discharging tube includes a first liquid discharging section, a bent section and a second liquid discharging section. A portion of the liquid discharging tube protrudes towards a direction away from the coupling part to form the bent section. The first liquid discharging section extends into and is fixed in the second coupling cavity, and the second liquid discharging section extends along the lengthwise direction of the handgrip. The bent section connects the first liquid discharging section and the second liquid discharging section.

The image tube according to the embodiment of the present application has a simple structure, and is adaptive in terms of length. The length of the image tube can be automatically adapted according to a length of a visual channel, in which the image tube is sheathed, of an endoscope fitting assembly, to realize an accurate and appropriate fitting between a tail end of the image tube and the visual channel, in which the image tube is sheathed, of the endoscope fitting assembly. In this way, it can solve the problem of reduced adaptability caused by a manufacturing tolerance and an assembly tolerance of the image tube and the visual channel, in which the image tube is sheathed, of the endoscope fitting assembly, and the problem of product consistency. Therefore, it facilitates the image tube to acquire a high quality image, the probability of damage to the image tube is lowered caused by a hard bump and collision between the image tube and the endoscope fitting assembly when the image tube is in insertion-extraction fit with the endoscope fitting assembly, the use cost is reduced, and the working efficiency of the image tube is improved.

### BRIEF DESCRIPTION OF THE DRAWINGS

FIG. 1 is a schematic diagram of an endoscope according to an embodiment of the present application.
FIG. 2 is a schematic exploded view of the endoscope shown in FIG. 1.
FIG. 3 is a schematic diagram of an image tube according to an embodiment of the present application.
FIG. 4 is a schematic exploded view of the image tube shown in FIG. 3.
FIG. 5 is a schematic cross-sectional view of the image tube shown in FIG. 3.
FIG. 6 is a schematic enlarged view of a portion A of FIG. 5.
FIG. 7 is a schematic diagram illustrating a fitting of internal structures of the endoscope shown in FIG. 1.
FIG. 8 is a schematic diagram illustrating a fitting of internal structures of the endoscope shown in FIG. 1 from another viewing angle.
FIG. 9 is a schematic cross-sectional view of the endoscope shown in FIG. 1.
FIG. 10 is a schematic enlarged view of a distal end of the endoscope shown in FIG. 9.
FIG. 11 is another schematic cross-sectional view of the endoscope shown in FIG. 1, in which an image tube is omitted.
FIG. 12 is a schematic cross-sectional view of a portion of the endoscope shown in FIG. 1.
FIG. 13 is another schematic cross-sectional view of a portion of the endoscope shown in FIG. 1.
FIG. 14 is a schematic diagram illustrating a fitting of a fixing support, a visual sleeve, a working channel tube, a cable and a light emitter shown in FIG. 2.
FIG. 15 is a schematic diagram of the portion shown in FIG. 12 from another viewing angle.
FIG. 16 is a schematic cross-sectional view illustrating a fitting of various components of a distal end of the endoscope shown in FIG. 1, in which a light emitter, a cable, a tube cover and a glue filling layer are omitted.
FIG. 17 is a schematic cross-sectional view illustrating a fitting of various components of a distal end of the endoscope shown in FIG. 1, in which a glue filling layer is omitted.
FIG. 18 is a schematic cross-sectional view of a distal end surface of the endoscope shown in FIG. 1.
FIG. 19 is a schematic diagram of an incident lens element and a reflecting prism shown in FIG. 2.
FIG. 20 is a schematic cross-sectional view of various components of a distal end of the endoscope shown in FIG. 1.
FIG. 21 is a schematic diagram illustrating a fitting of the image tube and an optical window shown in FIG. 2, in which arrows indicate a direction of external light.
FIG. 22 is an enlarged schematic cross-sectional view of a coupling part and a screw cap shown in FIG. 7.
FIG. 23 is a schematic cross-sectional view of a distal end of an image tube according to another embodiment of the present application.
FIG. 24 is a schematic cross-sectional view of a distal end of an image tube according to yet embodiment of the present application.
FIG. 25 is a schematic cross-sectional view of a distal end of an image tube according to yet another embodiment of the present application.

### DETAILED DESCRIPTION

It should be noted that the embodiments and the technical features of the embodiments of the present application may be combined with each other without conflict, and the description of the detailed description should be understood as an explanation of the present application and should not be construed as unduly limiting the present application.

In the description of the embodiments of the present application, the terms "inner", "outer" and the like for indicating orientation or positional relationship should be understood based on the orientation or positional relationship shown in the drawings. These terms are intended only for the convenience of describing the present application and simplifying the description, and are not intended to indicate or imply that the device or element must have a specific orientation, be constructed and operated in a specific orientation, and therefore cannot be interpreted as a restriction on the present application.

An embodiment of the present application provides an image tube 14, which includes a core tube 142 and an elastic member 146.

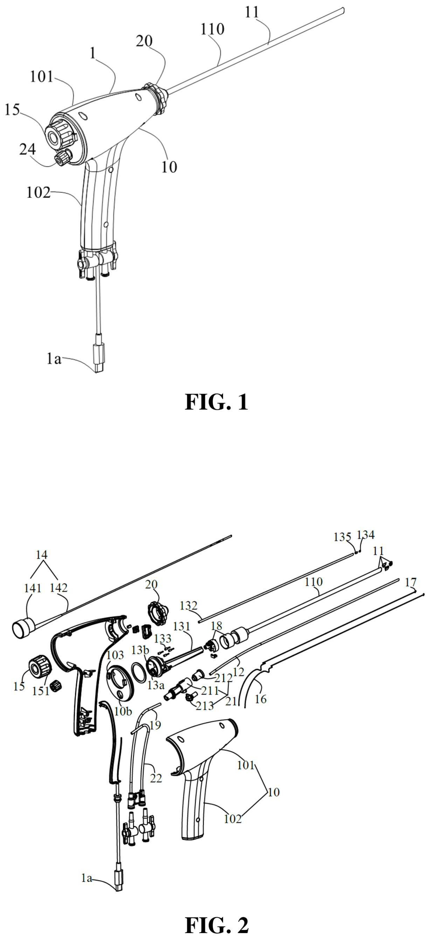

An embodiment of the present application provides an endoscope 1. Referring to FIG. 1 and FIG. 2, the endoscope 1 includes an endoscope fitting assembly and the image tube 14 according to any one of embodiments of the present application.

For convenience of description, an end extending into or close to an interior of a human body or an animal body during operation is referred to as a distal end, and an end held by an operator or close to an operator's hand is referred to as a proximal end.

The image tube 14 is configured to be in insertion-extraction fit with the endoscope fitting assembly to form the endoscope 1 for examination, diagnosis and surgical treatment of tissue inside the human body or the animal body.

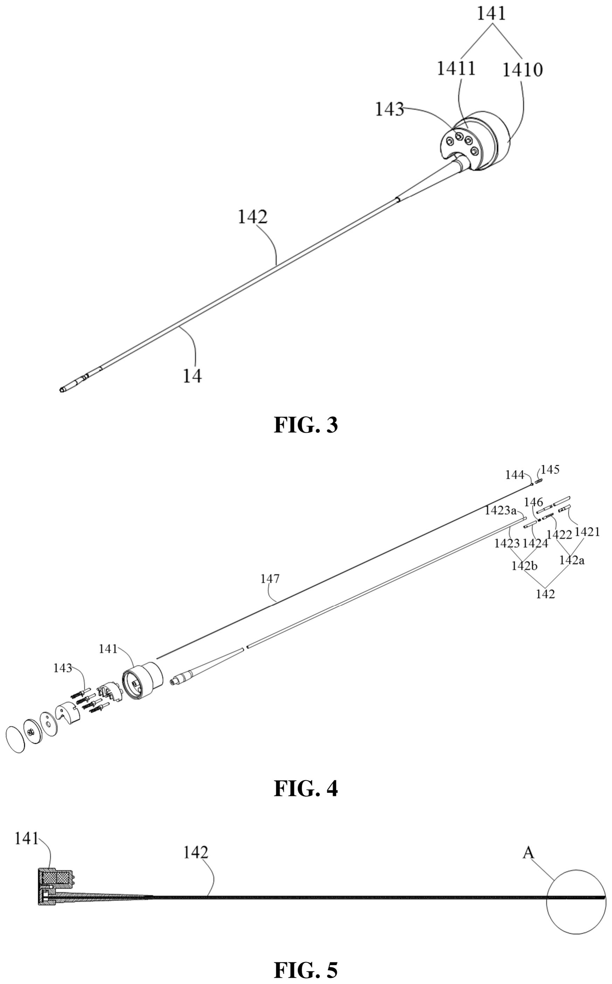

Referring to FIG. 3 and FIG. 4, the core tube 142 includes a main insertion section 142b and a head end section 142a in a lengthwise direction of the core tube 142. A proximal end of the head end section 142a is connected to a distal end of the main insertion section 142b. The head end section 142a and/or the main insertion section 142b is/are axially movable close to each other under an action of an external force.

The elastic member 146 is disposed in the core tube 142. The elastic member 146 is compressible and resilient, to actuate the head end section 142a producing the moving tendency which causes the head end section 142a to be axially away from the main insertion section 142b.

It should be noted that the head end section 142a and/or the main insertion section 142b being axially movable close to each other under the action of the external force includes multiple situations. In some embodiments, the head end section 142a is axially movable close to the main insertion section 142b under the action of the external force. In other embodiments, the main insertion section 142b is axially movable close to the head end section 142a under the action of the external force. In still other embodiments, the head end section 142a and the main insertion section 142b are axially movable close to each other under the action of the external force.

Specifically, when the image tube 14 is in insertion-extraction fit with the endoscope fitting assembly, and when a distal end surface of the head end section 142a is kept against an inner surface of a distal end of a visual channel, in which the image tube 14 is sheathed, of the endoscope fitting assembly, the head end section 142a is subjected to an external force from the inner surface of the distal end of the visual channel, in which the image tube 14 is sheathed, of the endoscope fitting assembly, and the head end section 142a axially moves close to the main insertion section 142b. At this moment, the elastic member 146 is compressed to generate compression deformation. After the image tube 14 is sheathed in and fitted with the visual channel, in which the image tube 14 is sheathed, of the endoscope fitting assembly, a resilient force of the elastic member 146 actuates the head end section 142a producing the moving tendency which causes the head end section 142a to axially be away from the main insertion section 142b. In this way, the distal end surface of the head end section 142a is kept against the inner surface of the distal end of the visual channel , in which the image tube 14 is sheathed, of the endoscope fitting assembly, thereby realizing a stable fitting between the image tube 14 and the endoscope fitting assembly.

It can be understood that there are following situations that a resilient force of the elastic member 146 actuates the head end section 142a producing the moving tendency which causes the head end section 142a to axially be away from the main insertion section 142b. In some situations, the head end section 142a does not axially move under the resilient force of the elastic member 146 and is stably fitted with the endoscope fitting assembly. In other situations, the head end section 142a axially moves away from the main insertion section 142b under the resilient force of the elastic member 146 and thus is stably fitted with the endoscope fitting assembly.

It can be understood that after the image tube 14 is sheathed in the endoscope fitting assembly, the head end section 142a is still subjected to the external force. After the image tube 14 is extracted out, the external force is revoked. After the image tube 14 is completely extracted out, the elastic member 146 resumes its initial state without deformation, and the head end section 142a is also reset to an initial position under an action of the elastic member 146. The initial position refers to a position in which the head end section 142a is not subjected to the external force.

It should be noted that a distal end of the endoscope is provided with an optical window configured to converge light and controllably change a propagation direction of an optical axis. The optical window may include a prism group, a concave lens group and the like. A distal end of the image tube is provided with a visual system, the visual system is further configured to process, converge and transmit the light of the optical window, and then convert an optical signal into an electrical signal for transmission. The visual system is a high-precision assembly. When the image tube is in insertion-extraction fit with the endoscope fitting assembly, the visual system should be accurately aligned with the optical window. The visual system cannot exert an excessive force on the optical window, and a length of the image tube is not allowed to exceed a length of the visual channel, in which the image tube is sheathed, of the endoscope fitting assembly, which causes the image tube to fail to fit with the endoscope fitting assembly.

Therefore, in the embodiments of the present application, the head end section 142a and/or the main insertion section 142b is/are axially movable close to each other under the action of the external force, and the elastic member 146 is compressible and resilient, to actuate the head end section 142a producing the moving tendency which causes the head end section 142a to axially be close to the main insertion section 142b. In this way, when the image tube 14 is in insertion-extraction fit with the endoscope fitting assembly, the image tube 14 is adaptive in terms of length, and the length of the image tube 14 may be automatically adapted to the length of the visual channel, in which the image tube 14 is sheathed, of the endoscope fitting assembly. In addition, a driving force can be provided for an axial alignment of the distal end surface of the head end section 142a and the optical window, to realize an accurate and appropriate fitting between the distal end surface of the head end section 142a and the optical window. Therefore, it is probable to lower the damage of the distal end surface of the head end section 142a and the visual channel, in which the image tube 14 is sheathed, of the endoscope fitting assembly due to the forcible fitting therebetween, it is probable to reduce the fitting gap between the head end section 142a and the visual channel, in which the image tube 14 is sheathed, of the endoscope fitting assembly arises from a lack of tight fitting therebetween, and it facilitates the image tube 14 to acquire a high quality image.

It can be understood that there are no limitations on the specific configuration of the elastic member 146 as long as the elastic member 146 can be compressed and generate a resilient force which actuates the head end section 142a producing the moving tendency which causes the head end section 142a to axially be away from the main insertion section 142b. For example, the elastic member 146 is a compression spring.

There are no limitations on the forming manner of the visual channel, in which the image tube 14 is sheathed, of the endoscope fitting assembly. In some examples, the endoscope fitting assembly includes a handle assembly 10, an insertion tube assembly 11 and a visual sleeve 13. The insertion tube assembly 11 includes an insertion tube 110, and a proximal end of the insertion tube 110 is connected to the handle assembly 10. The visual sleeve 13 is disposed inside the insertion tube 110, and the visual sleeve 13 is provided with a visual channel 130 for sheathing the image tube 14. In this embodiment, the endoscope fitting assembly is provided with the visual sleeve 13 for sheathing and fitting with the image tube 14, and the image tube 14 is in insertion-extraction fit with the visual sleeve 13. A distal end portion of the visual sleeve 13 is provided with a closed optical window. The core tube 142 is insertable into the visual channel 130, and a distal end portion of the head end section 142a is configured to be against an inner surface of the optical window.

It can be understood that in this embodiment, the optical window is formed at the distal end portion of the visual sleeve 13. The optical window is a component integrated with a plurality of optical elements, and can process the light from the tissue inside the human body or the animal body by means of converging and diverting, to allow the light to be transmitted into the visual channel 130 and to be received by the image tube 14. The image tube 14 may be kept against the inner surface of the optical window by being inserted into the visual sleeve 13.

Specifically, the core tube 142 is insertable into the visual channel 130, and the head end section 142a is axially movable close to the main insertion section 142b under the action of the external force. The elastic member 146 is compressed to lead to the compression deformation, and the resilient force of the elastic member 146 actuates the head end section 142a producing the moving tendency which causes the head end section 142a to axially be away from the main insertion section 142b, which also provides drive for the axial alignment of the head end section 142a and the optical window. A circumference of the head end section 142a is confined by an inner circumferential surface of the visual channel 130, to allow the distal end portion of the head end section 142a to be appropriately fitted against the inner surface of the optical window. In this way, a length of the core tube 142 can be adapted to the visual channel 130, the visual sleeve 13 can also protect the image tube 14, and it facilitates the image tube 14 to acquire the high-quality image.

The optical window may include a concave lens and a total internal reflecting prism, and is configured to converge the light and change a direction of the light, which facilitates the acquisition of the image by the image tube 14. The concave lens may be a plano-concave lens and the like.

The image tube 14 according to the embodiment of the present application has a simple structure, and is adaptive in terms of length. The length of the image tube 14 can be automatically adapted according to the length of the visual channel, in which the image tube 14 is sheathed, of the endoscope fitting assembly, to realize an accurate and appropriate fitting between the distal end of the image tube 14 and the visual channel, in which the image tube is sheathed, of the endoscope fitting assembly. When there is a large tolerance between the length of the image tube 14 and the length of the visual channel, in which the image tube 14 is sheathed, of the endoscope fitting assembly, the length of the image tube 14 can be shortened by the axial movement of the head end section 142a of the image tube 14 under the action of the external force, or the head end section 142a may be actuated by a resilient action of the elastic member 146 producing the moving tendency which causes the head end section 142a to be axially away from the main insertion section 142b and be kept against the inner surface of the distal end of the visual channel, in which the image tube 14 is sheathed, of the endoscope fitting assembly. In this way, it can solve the problem of worsened adaptability which is caused by a manufacturing tolerance or an assembly tolerance of the image tube and the visual channel, in which the image tube is sheathed, of the endoscope fitting assembly. Therefore, it facilitates the image tube 14 to acquire a high-quality image, it is probable to reduce damage to the image tube 14 caused by a hard bump and collision between the image tube 14 and the endoscope fitting assembly when the image tube 14 is in insertion-extraction fit with the endoscope fitting assembly, the use cost is reduced, and the working efficiency of the image tube 14 is improved.

It should be noted that the visual channel, in which the image tube 14 is sheathed, of the endoscope fitting assembly as described above can be referred to as the visual channel 130 mentioned below.

The handle assembly 10 is provided with a liquid inlet channel. It should be noted that the liquid inlet channel is a section of channel through which an external fluid passes through the handle assembly 10. The liquid inlet channel may be a channel formed by a tubular lumen, or may be a combination of a channel formed by a tubular lumen and a channel formed by other components.

A distal end surface of the insertion tube 110 is provided with a liquid outlet 110a.

It can be understood that an interior of the insertion tube 110 is provided with a free space in communication with the liquid inlet channel and the liquid outlet 110a. Herein, the free space is an unoccupied space in the insertion tube 110.

It can be understood that the external fluid flows to the human body or the animal body through the liquid inlet channel, the free space, and liquid outlet 110a, to inflate the observation region and allow the tissue inside the human body or the animal body to be flushed. The external fluid may be 5% glucose solution or normal saline.

The endoscope fitting assembly includes a working channel tube 12, and a working channel through which a surgical instrument passes is formed inside the working channel tube 12. The working channel tube 12 is arranged inside the insertion tube 110 and is provided with an outlet 12a at the distal end surface of the insertion tube 110.

The surgical instrument is inserted into the working channel tube 12 and extends into the human body or the animal body from the outlet 12a. There are no limitations on the specific type of the surgical instrument, for example, biopsy forceps, scissors, fixation drills, touch probes, hook forceps, electrodes and the like.

It should be noted that, in some application scenarios in which it is necessary to enter into the human body or the animal body through a natural lumen or a surgical stoma, as far as straight endoscopes without side-viewing functions or oblique-viewing functions are concerned, operators have to hold the handle assembly of the endoscope and acquire a suitable observation range or field of view by adjustment actions such as "raising" or "prying". However, on the one hand, the adjustment actions such as "raising" or "prying" require that the size of the natural lumen or the surgical stoma in human body or animal body should not be too small. On the other hand, such adjustment actions can easily cause damage to the human body or the animal body.

In the related art, a front end of the endoscope does not adopt optical designs with oblique-viewing functions, and its camera can only acquire a front image without a side-viewing function. The distal end of the endoscope is pre-bent to a certain angle to acquire a side image of the image tube, which facilitates the observation. However, the existence of the pre-bent angle leads to problems of inconvenient operation, inconvenient adjustment of a field of view and unsmooth manipulation of the instruments in use.

It can be understood that in order to facilitate the insertion tube to be inserted into the natural lumen or the surgical stoma of the human body or the animal body, an outer diameter of the insertion tube should be as small as possible. A plurality of channels such as the working channel tube, the liquid inlet channel and the like are disposed inside the insertion tube. Accordingly, there are strict limitations on a diameter of each of the plurality of channels. In the related art, the optical window of the endoscope is formed at a distal end portion of the image tube, and the optical window generally includes optical elements such as a lens, a prism and the like. If the image tube is disposed in the visual sleeve, a diameter of the optical window located at the distal end portion of the image tube is further compressed, and which obviously compress outer diameters of the optical elements such as a lens, a prism and the like for receiving the light inside the human body or the animal body. In this case, if it is necessary to solve the problem that observation viewing angles of the endoscope bring the inconvenience in surgery, and endow the optical window with side-viewing functions or oblique-viewing functions. That is, after the incident light passes through the optical window, a main optical axis changes a propagation direction according to the needs. In this way, there may be problems that an entrance pupil diameter of the optical window located at the distal end portion of the image tube becomes smaller, and the effective light received by the image tube is reduced.

In the embodiment of the present application, it is the straight endoscope 1, with which the adjustment actions like "raising" or "prying" are reduced greatly, and which facilitates the operator to acquire a preferable observation angle. A distal end of the endoscope 1 according to the embodiment of the present application is also provided with the optical window configured to converge the light and controllably change the propagation direction of the optical axis, and the optical window is disposed at a distal end of the visual sleeve 13. In a case that a diameter of the visual sleeve 13 and a diameter of the image tube 14 are unchanged, an effective diameter of the optical window can be increased, and further the entrance pupil diameter of the optical window can be increased, to allow the image tube 14 to receive more effective light, and facilitate outputting higher quality image. The diffuse reflection light in the tissue inside the human body or the animal body can change the propagation direction after being incident on an optical deflection assembly, to allow the endoscope 1 to have the side-viewing functions or the oblique-viewing functions.

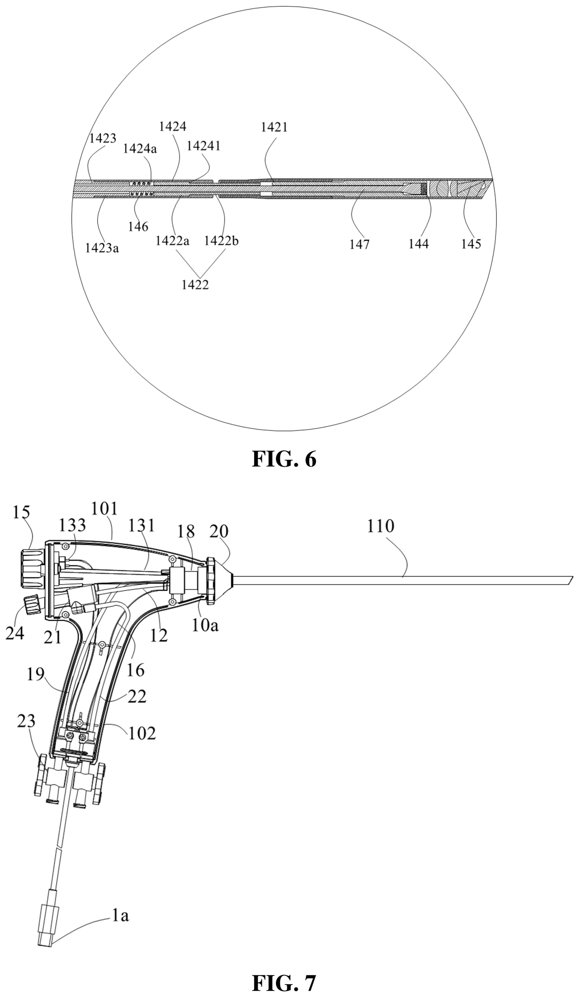

There are no limitations on the fitting manner of the image tube 14 and the visual sleeve 13. In some embodiments, referring to FIG. 7 to FIG. 10, the visual sleeve 13 is provided with a visual insertion-extraction port 13a at a proximal end of the handle assembly 10, and the image tube 14 is in detachable insertion-extraction fit with the visual channel 130 through the visual insertion-extraction port 13a. A distal end of the visual channel 130 is provided with a closed optical window at the distal end surface of the insertion tube 110, which protects the image tube 14 and facilitates the image tube 14 to acquire in vivo images of the human body or the animal body.

It should be noted that except for the visual insertion-extraction port 13a, the remaining portions of the visual channel 130 are closed.

In use, the image tube 14 can be directly inserted into the visual sleeve 13 or extracted out from the visual sleeve 13 through the visual insertion-extraction port 13a. In this way, the operation is simple, and the disassembly and the assembly are facilitated. The visual sleeve 13 can protect the image tube 14 to allow the image tube 14 to be not in contact with the tissue or liquid inside the human body or the animal body. Therefore, it's probable to reduce the cross-infection when the image tube 14 is reused, the disinfection level of the image tube 14, and the disinfection cost.

It should be noted that according to the requirements of relevant principles for medical disinfection and sterilization, a reusable instrument needs to undergo high-level disinfection after surgery if it is in contact with the tissue inside the human body or the animal body during surgery. If the surgical instrument is not in contact with the tissue inside the human body or the animal body, the disinfection level can be lowered, and only general disinfection such as wiping is enough. In the related art, since an end of the image tube is exposed to a distal end portion of the endoscope, the end of the image tube may be in contact with secretions and the like inside the human body or the animal body, and may be in contact with the tissue inside the human body or the animal body. Therefore, it is necessary to perform the high-level disinfection after surgery, such as immersion disinfection, high-temperature steam disinfection, ethylene oxide disinfection and the like.

There are no limitations on the materials of the handle assembly 10, the insertion tube 110, the working channel tube 12, and the visual sleeve 13. The insertion tube 110 may be a flexible tube or a rigid tube. The insertion tube 110, the working channel tube 12, the visual sleeve 13 and the like may be made of metal or plastic material. For example, the handle assembly 10 is made of plastic material, thus the use cost is low. The working channel tube 12, the visual sleeve 13, and the insertion tube 110 are made of steel tube, thus the strength is high and the weight is light.

The handle assembly 10, the insertion tube 110, the working channel tube 12 and the visual sleeve 13 can be used as disposable medical devices. The image tube 14 with high production cost can be reused, and can be directly removed from the visual sleeve 13 after use, and can be used again after low-level disinfection such as wiping.

In this embodiment, since the visual sleeve 13 can protect the image tube 14, the image tube 14 is not in contact with the human body or the animal body, and is not in contact with the liquid entering into the endoscope 1. In this way, it is prevented from the cross-infection-caused by incomplete cleaning and disinfection, and it's probable to reduce blurring images caused by damage to a front end lens of the image tube due to excessive disinfection times in the related art. The image tube 14 can be reused, and can be used again after being disinfected by the low-level disinfection such as wiping in a post-operative disinfection process without the high-level disinfection such as immersion in disinfectant, which is convenient, quick and low in use cost.

There are no limitations on the specific configuration of the head end section 142a and the main insertion section 142b, and also no limitations on the manner in which the connection is realized.

In some embodiments, referring to FIG. 4, FIG. 5, and FIG. 6, the head end section 142a includes a first body tube 1421 and a first casing tube 1422. The first casing tube 1422 is provided as a separate component and fixed to a proximal end of the first body tube 1421. The main insertion section 142b includes a second body tube 1423 and a second casing tube 1424. The second casing tube 1424 is provided as a separate component and fixed to a distal end of the second body tube 1423. The head end section 142a and the main insertion section 142b are connected to each other through the first casing tube 1422 and the second casing tube 1424.

The first body tube 1421 can support the first casing tube 1422, and the second body tube 1423 can support the second casing tube 1424. In this way, it can improve the stability of the connection between the head end section 142a and the main insertion section 1424, and there is no need of increasing an axial length, an outer diameter of each of the first casing tube 1422, and the second casing tube 1424, as long as the connection can be realized. It's unnecessary to increase the outer diameter of the second casing tube 1424 which may be substantially the same as the outer diameter of the first body tube 1421, and a mounting space of the image tube 14 is reduced. In this way, it facilitates the insertion of the image tube 14 into the visual channel, in which the image tube 14 is sheathed, of the endoscope fitting assembly, and the insertion-extraction fit of the image tube 14 and the endoscope fitting assembly, specifically, it facilitates the insertion of the image tube 14 into the visual channel 130.

There are no limitations on the materials of the first body tube 1421, the first casing tube 1422, the second body tube 1423, and the second casing tube 1424. For example, the first body tube 1421, the first casing tube 1422, the second body tube 1423, and the second casing tube 1424 are made of stainless steel, with the high processing accuracy, the high material strength, and corrosion resistance.

There are no limitations on the fitting manner of the second casing tube 1424 and the second body tube 1423. For example, the second casing tube 1424 may be arranged around the second body tube 1423, or the second body tube 1423 may be arranged around the second casing tube 1424. There are no limitations on the arrangement manner of the first casing tube 1422 and the second casing tube 1424. For example, the first casing tube 1422 may be arranged around the second casing tube 1424, or the second casing tube 1424 may be arranged around the first casing tube 1422, as long as the length of the image tube 14 can be adaptively adjustable.

In some embodiments, referring to FIG. 6, a first dwindling section 1423a is formed at the distal end of the second body tube 1423. The first dwindling section 1423a is inserted inside a proximal end of the second casing tube 1424, and the first casing tube 1422 is arranged inside a distal end of the second casing tube 1424.

One end of the elastic member 146 is kept against a distal end portion of the second body tube 1423, and another end of the elastic member 146 is kept against a proximal end portion of the first casing tube 1422.

Referring to FIG. 6, in this embodiment, the elastic member 146 is arranged inside the second casing tube 1424, and each of two ends of the elastic member 146 is kept against a respective one of the second body tube 1423 and the first casing tube 1422. In this way, it's more reliable to fix the elastic member 16, and it increases the reliability of the length adjustment of the image tube 14.

In other embodiments, a second dwindling section is formed at a proximal end of the second casing tube 1424. The second dwindling section is inserted inside a distal end of the second body tube 1423, and the first casing tube 1422 is arranged around a distal end of the second casing tube 1424.

One end of the elastic member 146 is kept against a distal end portion of the second body tube 1423, and another end of the elastic member 146 is kept against a proximal end portion of the first casing tube 1422.

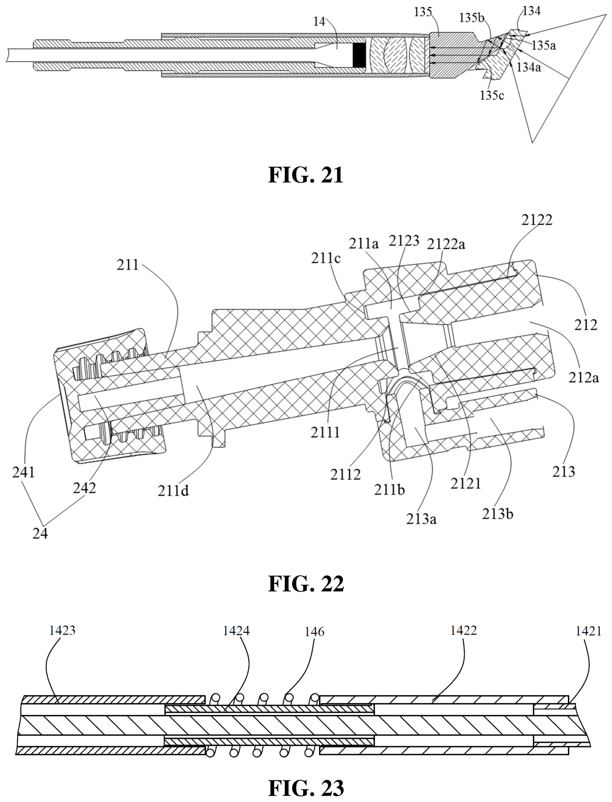

In this embodiment, referring to FIG. 23, it can be understood that the elastic member 146 is arranged around the second casing tube 1424, and each of two ends of the elastic member 146 is kept against a respective one of the second body tube 1423 and the first casing tube 1422.

Specifically, in the two embodiments as described above, each of two ends of the elastic member 146 fits with a respective one of the second body tube 1423 and the first casing tube 1422. When the image tube 14 is in insertion-extraction fit with the visual sleeve 13, the first body tube 1421 is subjected to an external force from the inner surface of the optical window located at the distal end of the visual sleeve 13 to the second body tube 1423, to further push the first casing tube 1422 to axially move close to the second body tube 1423. Thus, the elastic member 146 is compressed and deformed. In addition, the first casing tube 1422 is also subjected to the resilient force generated from the elastic member 146, the resilient force actuates the first casing tube 1422 producing the moving tendency which causes the first casing tube 1422 to axially be away from the second body tube 1423, then a distal end of the head end section 142a is kept against and fit with the inner surface of the optical window reliably.

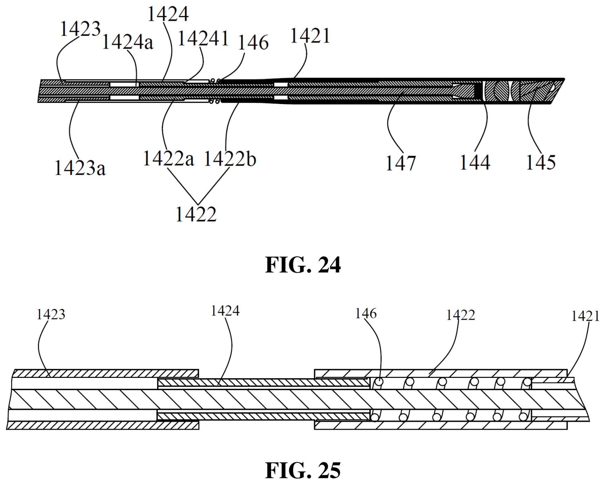

In some embodiments, referring to FIG. 24, it can be understood that the distal end of the second body tube 1423 is inserted into the proximal end of the second casing tube 1424, and the first casing tube 1422 is arranged inside the distal end of the second casing tube 1424. The elastic member 146 is arranged around the first casing tube 1422 and is located between a distal end portion of the second casing tube 1424 and a proximal end portion of the first body tube 1421.

In other embodiments, referring to FIG. 25, the proximal end of the second casing tube 1423 is inserted into the distal end of the second body tube 1424, and the first casing tube 1422 is arranged around the distal end of the second casing tube 1424. The elastic member 146 is arranged inside the first casing tube 1422 and is located between a distal end portion of the second casing tube 1424 and a proximal end portion of the first body tube 1421.

In the two embodiments that the elastic member 146 is located between the distal end of the second casing tube 1424 and the proximal end of the first body tube 1421, when the image tube 14 is in insertion-extraction fit with the visual sleeve 13, the first body tube 1421 is subjected to the external force, to further push the first casing tube 1422 to axially move close to the second body tube 1423. Thus, the elastic member 146 is compressed and deformed. In addition, the first body tube 1421 is also subjected to the resilient force generated from the elastic member 146, the resilient force actuates the first casing tube 1422 producing the moving tendency which causes the first casing tube 1422 to axially be away from the second body tube 1423, then the distal end of the head end section 142a is kept against and fit with the inner surface of the optical window reliably.

In some embodiments, referring to FIG. 6, the second casing tube 1424 is provided with an accommodating lumen 1424a in which the first dwindling section 1423a is accommodated. The first casing tube 1422 includes a first casing section 1422a and a second casing section 1422b in a lengthwise direction of the first casing tube 1422, and an outer diameter of the first casing section 1422a is greater than an outer diameter of the second casing section 1422b. The first casing section 1422a is inserted into the accommodating lumen 1424a, and a distal side wall of the accommodating lumen 1424a protrudes inwardly to form a limiting wall 14241. A proximal end portion of the first casing section 1422a is kept against the elastic member 146, and a distal end portion of the first casing section 1422a detachably abuts against the limiting wall 14241.

The proximal end portion of the first casing section 1422a is kept against the elastic member 146, and the distal end portion of the first casing section 1422a is confined by the limiting wall 14241. That is, the axial movement of the first casing section 1422a is confined in the accommodating lumen 1424a, and the accommodating lumen 1424a has the protection functions for the movement of the first casing section 1422a. In this way, there are no affections on the movement of the first casing section 1422a and the elastic member 146 from other components, it can avoid scratching and increase the stability of the image tube 14.

In some embodiments, referring to FIG. 6, a circumferential outer side wall of the first casing section 1422a is tangent to a side wall of the accommodating lumen 1424a, which facilitates the movement of the first casing section 1422a in the accommodating lumen 1424a. In this way, the movement of the first casing section 1422a is smoother and easier, with resistance reduced during the movement. It facilitates the alignment and fitting of the distal end portion of the head end section 142a and the distal end of the visual channel, in which the image tube 14 is sheathed, of the endoscope fitting assembly, and in particular, it facilitates the alignment and fitting of the distal end portion of the head end section 142a and the optical window of the distal end of the vision sleeve 13.

In some embodiments, referring to FIG. 6, the second casing tube 1424 is arranged around the first casing tube 1422, and the distal end portion of the second casing tube 1424 is axially spaced apart from the proximal end portion of the first body tube 1421 to define a movement stroke of the head end section 142a in an axial direction.

Under the action of the external force, the head end section 142a is axially movable close to the main insertion section 142b, and the movement stroke of the head end section 142a is confined by a structure in which the distal end portion of the second casing tube 1424 is axially spaced apart from the proximal end portion of the first body tube 1421.

Under the resilient force of the elastic member 146, there is the tendency which causes the head end section 142a to be axially away from the main insertion section 142b, and the movement stroke of the head end section 142a is confined by a distance between the distal end portion of the first casing section 1422a and the limiting wall 14241 of the second casing section 1424.

That is, when the head end section 142a axially moves close to the main insertion section 142b under the action of the external force, the structure in which the distal end portion of the second casing tube 1424 is spaced apart from the proximal end portion of the first body tube 1421 confines the movement displacement of the head end section 142a. When the head end section 142a axially moves away from the main insertion section 142b under the resilient force of the elastic member 146, an axial distance between the distal end portion of the first casing section 1422a and the limiting wall 14241 of the second casing section 1424 confines the movement stroke of the head end section 142a, to allow the head end section 142a to be limited and protected. In this way, it can be prevented from damaging the head end section 142a and the visual channel, in which the image tube 14 is sheathed, of the endoscope fitting assembly, and in particular, it can avoid damaging the distal end of the image tube 14 and the optical window.

In other embodiments, the first casing tube 1422 is arranged around the second casing tube 1424, and the proximal end portion of the first casing tube 1422 is axially spaced apart from the distal end portion of the second body tube 1423 to define a movement stroke of the head end section 142a in an axial direction.

Under the action of the external force, the head end section 142a may axially move close to the main insertion section 142b, and the movement stroke of the head end section 142a is confined by a structure that the proximal end portion of the first casing tube 1422 is axially spaced apart from the distal end portion of the second body tube 1423.

Under the resilient force of the elastic member 146, there is the tendency which causes the head end section 142a to be axially away from the main insertion section 142b, and the movement stroke of the head end section 142a is confined by the distance between the distal end portion of the first casing section 1422a and the limiting wall 14241 of the second casing section 1424.

That is, when the head end section 142a axially moves close to the main insertion section 142b under the action of the external force, the structure in which the proximal end portion of the first casing tube 1422 is spaced apart from the distal end portion of the second body tube 1423 confines the movement displacement of the head end section 142a. When the head end section 142a axially moves away from the main insertion section 142b under the resilient force of the elastic member 146, an axial distance between the distal end portion of the first casing section 1422a and the limiting wall 14241 of the second casing section 1424 confines the movement stroke of the head end section 142a, to allow the head end section 142a to be limited and protected. In this way, it can be prevented from damaging the head end section 142a and the visual channel, in which the image tube 14 is sheathed, of the endoscope fitting assembly, and in particular, it can avoid damaging the distal end of the image tube 14 and the optical window.

It can be understood that there are no limitations on the fixing manner of the first body tube 1421 and the first casing tube 1422, and no limitations on the fixing manner of the second body tube 1423 and the second casing tube 1424, such as gluing, welding and the like.

For instance, the welding is used for the fitting between the first body tube 1421 and the first casing tube 1422, and also for the fitting between the second body tube 1423 and the second casing tube 1424. It can be described briefly to complete the assembly process of the head end section 142a and the main insertion section 142b in an embodiment of the present application with reference to FIG. 6.

In assembly process, the first casing tube 1422 extends into the second casing tube 1424, one portion of the first casing tube 1422 is retained in the second casing tube 1424, another portion of the first casing tube 1422 extends out of the distal end of the second casing tube 1424 and extends into the proximal end of the first body tube 1421, and the first body tube 1421 is fixed to the structure of the first casing tube 1422 extending into the first body tube 1421 by welding. In this way, the first body tube 1421 and the first casing tube 1422 form the head end section 142a, and a portion of the first casing tube 1422 that is not welded to the first body tube 1421 is axially movable. It can be understood that a gap is reserved between the proximal end portion of the first body tube 1421 and the distal end portion of the second casing tube 1424 to define the movement stroke of the head end section 142a. The elastic member 146 is disposed in the second casing tube 1424, the first dwindling section 1423a located at the distal end of the second body tube 1423 extends into the second casing tube 1424, and the second casing tube 1424 is welded to the first dwindling section 1423a. In this way, the second body tube 1423 and the second casing tube 1424 form the main insertion section 142b.

There are no limitations on the specific configuration of the optical window. In some embodiments, referring to FIG. 2 and FIG. 19 to FIG. 21, the optical window includes an incident lens element 134, and a reflecting prism 135 disposed in the visual sleeve 13. The incident lens element 134 is disposed at an end of the visual sleeve 13 and located on the light incident side of the reflecting prism 135. The incident lens element 134 is configured to receive external light, and refract and converge the external light to the reflecting prism 135, and the reflecting prism 135 is configured to transmit the light to a light incident portion of the image tube 14 in the visual channel 130 by reflection.

The incident lens element 134 may be an element integrated by combination of one or more concave lenses. Specifically, the incident lens element may be an element integrated by combination of one or more plano-concave lenses. The incident lens element 134 has a light converging function and enlarges an observation field of view of the endoscope 1.

When the endoscope 1 is operated, the light reflected by observed regions of the human body or the animal body is incident on the incident lens element 134. The incident lens element 134 refracts and converges the received light to the reflecting prism 135, and the reflecting prism 135 transmits the light to the light incident portion of the image tube 14 by reflection, to allow the image tube 14 to acquire optical signals. The incident lens element 134 is disposed at the end of the visual sleeve 13, and the reflecting prism 135 is disposed in the visual sleeve 13, which facilitates the image tube 14 to acquire a good observation angle, and makes full use of a space inside the visual sleeve 13. The entrance pupil diameter can be enlarged in case that a size of the visual sleeve 13 and a size of the insertion tube 110 are not enlarged, that is, the diameter of the visual sleeve 13 remains constant. In this way, an important prerequisite is provided for the acquisition of high quality optical signals by the image tube 14, which increases the working efficiency of the image tube 14.

It should be noted that in a projection of the image tube 14 toward the reflecting prism 135, a projection of the image tube 14 falls into the projection of the reflecting prism 135, that is, a contour of the light incident portion of the image tube 14 is located within a contour range of a light emitting portion of the reflecting prism 135, that is, the diameter of the reflecting prism 135 is greater than the diameter of the optical component inside the image tube 14. Accordingly, under this circumstance the diameter of the visual sleeve 13 and the diameter of the image tube 14 are kept constant with the diameter of the optical window increased, that is, it increases the diameter of the incident lens element 134 and the diameter of the reflecting prism 135, thereby increasing an effective coupling region of the incident lens element 134 and the reflecting prism 135, and increasing the entrance pupil diameter. The incident lens element 134 and the reflecting prism 135 can acquire a wider range of light reflected by the observed region of the human body or the animal body, and converge and transmit the light from wider range into the image tube 14, and it increases the effective light which can be received by the optical component inside the image tube 14, such that it's probably for the image tube 14 to acquire external images with higher quality.

It can be understood that when the assembly is performed, the incident lens element 134 and the reflecting prism 135 may be snap-fitted to each other, and then may be fixedly connected to an inner side wall of the visual channel 130 by a bonding manner. The distal end of the visual sleeve 13 is sealed by the reflecting prism 135 and the incident lens element 134, to allow the space inside the visual sleeve 13 to be completely isolated from a space outside the visual sleeve 13, and allow the image tube 14 inserted-extracted into the visual channel 130 to be completely isolated from the tissue and liquid inside the human body or the animal body. In this way, it facilitates the image tube 14 to acquire the external images.

In some embodiments, a glue filling layer may be disposed in a region between an inner wall of the insertion tube 110 and a circumference of the concave lens 134, such that the incident lens element 134 can be bonded and fixed further. In this way, the incident lens element 134 is prevented from accidentally falling off, the sealing effect of the visual channel 130 is ensured, the liquid inside the human body or the animal body is prevented from entering into the visual sleeve 13, and the working reliability of the image tube 14 is increased.

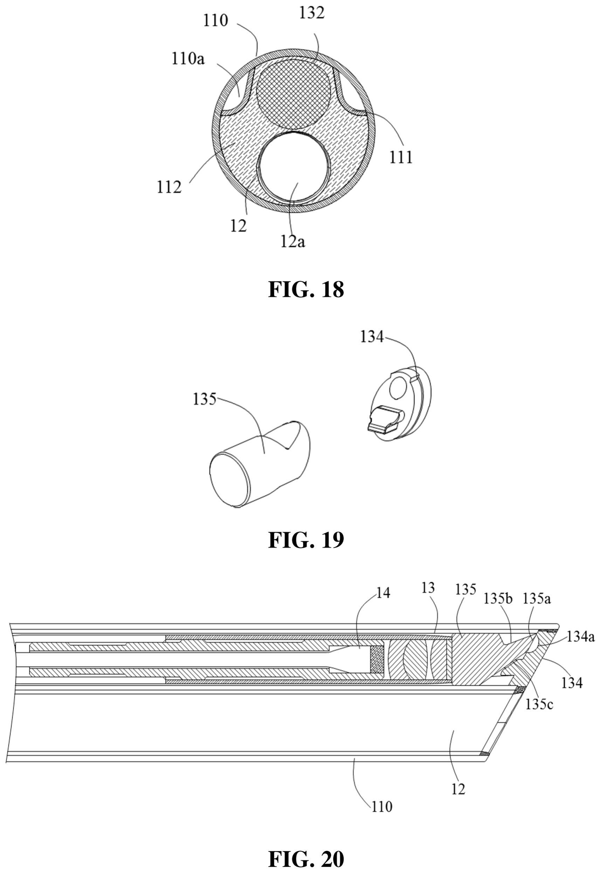

In some embodiments, referring to FIG. 20 and FIG. 21, a light emitting surface of the reflecting prism 135 is perpendicular to a lengthwise direction of the visual sleeve 13.

Referring to FIG. 20, a distal end surface of the image tube 14 is formed as a vertical surface, and the light emitting surface of the reflecting prism 135 is perpendicular to the lengthwise direction of the visual sleeve 13, that is, the light emitting surface of the reflecting prism 135 is also formed as a vertical surface. In this way, it facilitates the tight fitting between the distal end surface of the image tube 14 and the light emitting surface of the reflecting prism 135, and further facilitates the image tube 14 to acquire the high quality images.

In some embodiments, referring to FIG. 20 and FIG. 21, a distal end surface of the incident lens element 134 is an inclined surface, and an arc-shaped groove 134a is formed at a side of the incident lens element 134 facing toward the reflecting prism 135. A surface of the arc-shaped groove 134a is a light emitting surface of the concave lens 134, and a light incident surface 135a of the reflecting prism 135 covers the arc-shaped groove 134a.

It can be understood that the arc-shaped groove 134a is a coupling region between the incident lens element 134 and the reflecting prism 135.

The distal end surface of the incident lens element 134 is the inclined surface, that is, a distal end surface of the visual sleeve 13 is the inclined surface, which can enlarge the observation range and facilitate the acquisition of the image by the image tube 14. The operator can reduce the adjustment actions such as "raising" or "prying" on the endoscope 1, and can acquire the suitable observation angle.

The light incident surface 135a of the reflecting prism 135 covers the arc-shaped groove 134a. The diffuse reflection light from the tissue inside the human body or the animal body is incident on the incident lens element 134, and the incident lens element 134 can refract, converge and transmit the incident light to the light incident surface 135a of the reflecting prism 135, such that the incident light is further reflected and received by the image tube 14. In this way, it's probable to reduce the loss of light and increase the working reliability of the endoscope 1.

The reflecting prism 135 may be a total reflecting prism, and tansmit the light to the image tube 14 by total reflection without loss, which further increases the effective light that can be received by the image tube 14.

There are no limitations on the manner in which the reflecting prism 135 can realize the reflection. In some embodiments, referring to FIG. 20 and FIG. 21, the reflecting prism 135 is provided with at least a first reflecting surface 135b and a second reflecting surface 135c disposed face to face. The first reflecting surface 135b is inclined toward the arc-shaped groove 134a, and the second reflecting surface 135c is located on the light emitting side of the first reflecting surface 135b, to allow light emitted from the arc-shaped groove 134a to be emitted after being reflected by the first reflecting surface 135b and the second reflecting surface 135c in sequence, or to be emitted after being subjected to one or more light processing.

It can be understood that the first reflecting surface 135b and the second reflecting surface 135c may be optically coated with a film, that is, an outer surface of the reflecting prism 135 corresponding to the first reflecting surface 135b and the second reflecting surface 135c may be optically coated with a film. In this way, the reflection function can be realized on the first reflecting surface 135b and the second reflecting surface 135c.

Specifically, the light from the observed region of the human body or the animal body is incident on the incident lens element 134, refracted and converged to the first reflecting surface 135a through the arc-shaped groove 134a, and then emitted after being reflected to the second reflecting surface 135c through the first reflecting surface 135b, or received by the image tube 14 after being subjected to one or more light processing. The light processing may be reflection, convergence, scattering and the like.

It should be noted that the first reflecting surface 135b and the second reflecting surface 135c are disposed face to face, and the first reflecting surface 135b is inclined toward the arc-shaped groove 134a, that is, the second reflecting surface 135c is disposed away from the arc-shaped groove 134a. In this way, the light emitted by the arc-shaped groove 134a must be reflected by the first reflecting surface 135b before being received by the second reflecting surface 135c.

It can be understood that the first reflecting surface 135b and the second reflecting surface 135c are arranged inclined to the lengthwise direction of the visual sleeve 13. In this way, the light from the observed region of the human body or the animal body can be diverted by reflection after being refracted and converged by the incident lens element 134, and can be received by the image tube 14 in a manner close to the parallel light. Therefore, it's probable to reduce that the light from the observed region of the human body or the animal body enters a reception blind area of the image tube 14, reduce the light loss, and increase the working reliability of the incident lens element 134 and the reflecting prism 135. In addition, the endoscope 1 can change the viewing angle by rotating the insertion tube 110, to facilitate the side-viewing functions of the endoscope 1, such that it can improve the working efficiency of the image tube 14 and facilitate the examination and surgery.

In some embodiments, referring to FIG. 4 to FIG. 6, the distal end of the head end section 142a is integrated with an optical component 145, and a photoelectric conversion component 144 for converting an optical signal of the optical component 145 into an electrical signal. The image tube 14 includes a signal line 147 disposed in the core tube 142 and extending along the lengthwise direction of the core tube 142. A distal end of the signal line 147 is connected to the photoelectric conversion component 144.

The optical component 145 may be one or more lenses configured to converge and transmit the light to the photoelectric conversion component 144.

The photoelectric conversion component 144 is configured to convert the optical signal of the optical component 145 into an image electrical signal. The photoelectric conversion component 144 may be a Charge coupled Device, CCD, or a Complementary Metal Oxide Semiconductor, CMOS, or may be of other types, which are not limited herein.

It can be understood that the photoelectric conversion component 144 acquires the optical signal, generates the image signal, and transmits the image signal. In addition, the endoscope 1 may process an original image signal by an image processing board, such as denoise the image, correct a wide-angle distortion, improve a contrast, eliminate a red eye phenomenon and the like, to allow the quality of the image to be better, and then may transmit the processed image. There are no limitations on an arrangement position of the image processing board, and the image processing board may be arranged on the image tube 14, arranged inside the handle assembly 10, or arranged on an external device, as long as the image processing board can process the image. In some embodiments, a dimension of the image tube 14 is small, a signal transmission path is relatively short, and the image processing board may be arranged on the external device.

There are no limitations on the signal transmission manner between the image tube 14 and the external device, and it may be wired transmission or wireless transmission.

In some embodiments, referring to FIG. 2 to FIG. 5, the image tube 14 includes a disc part 141 connected to a proximal end of the core tube 142. The disc part 141 includes a disc body 1410 and a protrusion 1411 protruding in a direction from the disc body 1410 to the head end section 142a. A distal end of the protrusion 1411 is provided with a plurality of first electrical terminals 143, and a proximal end of the signal line 147 is electrically connected to the plurality of first electrical terminals 143.

In some embodiments, referring to FIG. 7 to FIG. 9, the disc part 141 is disposed to cover a proximal end portion of the visual sleeve 13, and the disc part 141 includes the disc body 1410 and the protrusion 1411 protruding from the disc body 1410 toward the visual sleeve 13. The plurality of first electrical terminals 143 is integrated on a side of the protrusion facing toward the visual sleeve 13. A conductive path is established between the plurality of first electrical terminals 143 and the photoelectric conversion component 144, and the electrical signal converted by the photoelectric conversion component 144 is transmitted to the plurality of first electrical terminals 143 through the signal line 147.

The visual sleeve 13 is provided with a plurality of second electrical terminals 133. Each of the plurality of first electrical terminals 143 is detachably connected to and conductively connected to a respective one of the plurality of second electrical terminals 133. The endoscope fitting assembly includes a data exchange interface 1a configured to interact with the external device, and the data exchange interface 1a is in electrical communication with the plurality of second electrical terminals 133.

When the endoscope 1 is operated, the light from the observed region of the human body or the animal body is converged and transmitted to the optical component 145 through the optical window, and the optical component 145 further processes and converges the transmitted light and then transmits the light to the photoelectric conversion component 144. The photoelectric conversion component 144 converts the optical signal into an image electrical signal. The image electrical signal is transmitted to each first electrical terminal 143 and each second electrical terminal 133 through the signal line 147, and then transmitted to the data exchange interface 1a. The image can be displayed on the external device, thereby acquiring a clear image of the observed tissue for diagnosis, treatment and corresponding surgical operation. There are no limitations on the connection manner of each of the plurality of first electrical terminals 143 and a respective one of the plurality of second electrical terminals 133.

Referring to FIG. 7 to FIG. 9, a proximal end of the visual sleeve 13 extends out of a proximal end portion of the handle assembly 10. The proximal end portion of the visual sleeve 13 is provided with a recess 13b and the visual insertion-extraction port 13a, and the proximal end portion of the visual sleeve 13 is recessed toward a direction close to the insertion tube 110 to form the recess 13b. The plurality of second electrical terminals 133 are disposed at a distal end of the recess 13b and penetrate through a groove wall of the recess 13b along an insertion-extraction direction of the image tube 14.

One end of each second electrical terminal 133 is exposed in the recess 13b, and conductively cooperates with a respective first electrical terminal 143. Another end of each second electrical terminal extends into a space in the handle assembly 10, and is electrically connected to the data exchange interface 1a through a connection wire. The protrusion 1411 extends into and is fixed in the recess 13b. That is, each of the plurality of first electrical terminals 143 is conductively connected to a respective one of the plurality of second electrical terminals 133 along the insertion-extraction direction of the image tube 14.

In this way, the plurality of second electrical terminals 133 does not affect the insertion-extraction fitting between the image tube 14 and the visual sleeve 13, and the connection between the plurality of second electrical terminals 133 and the data exchange interface 1a also does not need the plurality of second electrical terminals 133 to be in direct contact with the image tube 14.

It should be noted that an insertion orientation of the image tube will also affect the acquisition of the image of the image tube 14, and if the image tube is rotated or deflected during insertion, it will be affected to acquire the image of a normal orientation by the image tube 14. Therefore, in the embodiment of the present application, the protrusion 1411 of the image tube 14 is fitted with the recess 13b of the visual sleeve 13, which can play a positioning and orientating role when the image tube 14 is inserted into the visual sleeve 13. Therefore, it increases the reliability of insertion of the image tube 14, and the image tube 14 is prevented from being inserted into the visual sleeve 13 with any rotation angle, the insertion with any rotation angle may affect imaging.

It can be understood that a circumferential surface of the protrusion 1411 has a certain taper, and a cross-sectional area of the protrusion 1411 gradually decreases along a direction close to the recess 13b. A portion of the recess 13b corresponding to the protrusion 1411 also has a certain taper, and a cross-sectional area of the recess 13b also gradually decreases along a direction from a proximal end to a distal end of the recess 13b. In this way, when the protrusion 1411 is fitted with the recess 13b of the visual sleeve 13, a tapered structure of the protrusion 1411 and the recess 13b can facilitate the insertion-extraction of the image tube 14. The circumferential surface of the protrusion 1411 can be closely fitted against an inner wall of the recess 13b, which facilitates positioning and orientating of the image tube 14 and prevents the image tube 14 from being rotated at any angle. In addition, when the image tube 14 is inserted into the visual sleeve 13, the image tube 14 can be positioned and fixed by the protrusion 1411 together with the head end section 142a and the main insertion section 142b located at the distal end of the image tube 14, which further increases the positioning reliability of the image tube 14.

It can be understood that both the protrusion 1411 and the recess 13b have a certain taper, and/or other structures located at the proximal end of the visual sleeve 13 and at a proximal end of the image tube 14 have a certain taper, to allow the image tube 14 to be positioned when the image tube 14 is assembled. For example, the image tube 14 is inserted into the visual channel 130 under the action of the external force, and the elastic member 146 is compressed and contracted. When the protrusion 1411 is closely fitted with the recess 13b, the protrusion 1411 can no longer move toward the distal end under the action of the external force, and the proximal end of the image tube 14 can be circumferentially and axially positioned under the fitting between the protrusion 1411 and the recess 13b. However, when the external force cannot push the image tube 14 forward, the elastic member 146has an elastic potential energy thereof, thereby pushing the head end section 142a to be adaptively fitted against the optical window. The fitting between the protrusion 1411 and the recess 13b can also reduce the probability of damage to the image tube 14 and the optical window caused by excessive insertion of the image tube 14 into the visual sleeve 13, and increase the reliability of the length adaptive adjustment of the image tube 14.

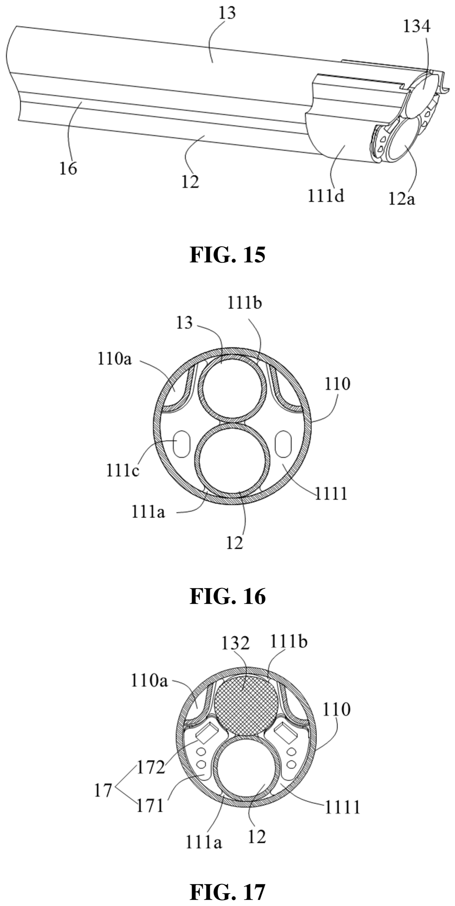

There are no limitations on the shape of the protrusion 1411. For example, referring to FIG. 15, a cross section of the protrusion 1411 is substantially crescent-shaped. When the image tube 14 is in insertion-extraction fit with the visual sleeve 13, the protrusion 1411 can be fitted with the recess 13b to play the positioning and orientating role without affecting the insertion of the core tube 142 into the visual sleeve 13.

There are no limitations on the fitting manner of the image tube 14 and the handle assembly 10, such as snapping, screwing and the like.

For example, referring to FIG. 7 to FIG. 9, the endoscope fitting assembly includes an end cap 15. A proximal end surface of the handle assembly 10 is provided with a first snapping part 103, the end cap 15 is arranged to cover around the disc part 141, that is, the disc part 141 is accommodated inside the end cap 15, and a circumferential surface of the end cap 15 is provided with a second snapping part 151. The first snapping part 103 is detachably snapped to the second snapping part 151, to allow the disc part 141 to be kept against the proximal end portion of the visual sleeve 13.

Specifically, the image tube 14 is inserted into the visual sleeve 13, the disc part 141 is located on a side of the proximal end of the visual sleeve 13, and the end cap 15 is arranged to cover around the disc part 141. The first snapping part 103 is snap-fitted with the second snapping part 151 by rotating the end cap 15. An end surface of the end cap 15 exerts a force toward the distal end on the disc part 141, to allow the disc part 141 to be kept against the end of the visual sleeve 13, and to prevent the image tube 14 from falling off or loosening.

The first snapping part 103 and the second snapping part 151 allow the disassembly and assembly of the handle assembly 10 and the image tube 14 to be more easier, which facilitates the operation of medical staff, improves the convenience of operation, and saves the preparation time of surgery. In addition, there is no need to provide a connecting structure on the image tube 14 for connecting to the handle assembly 10, to allow a structure of the disc part 141 to be simpler.

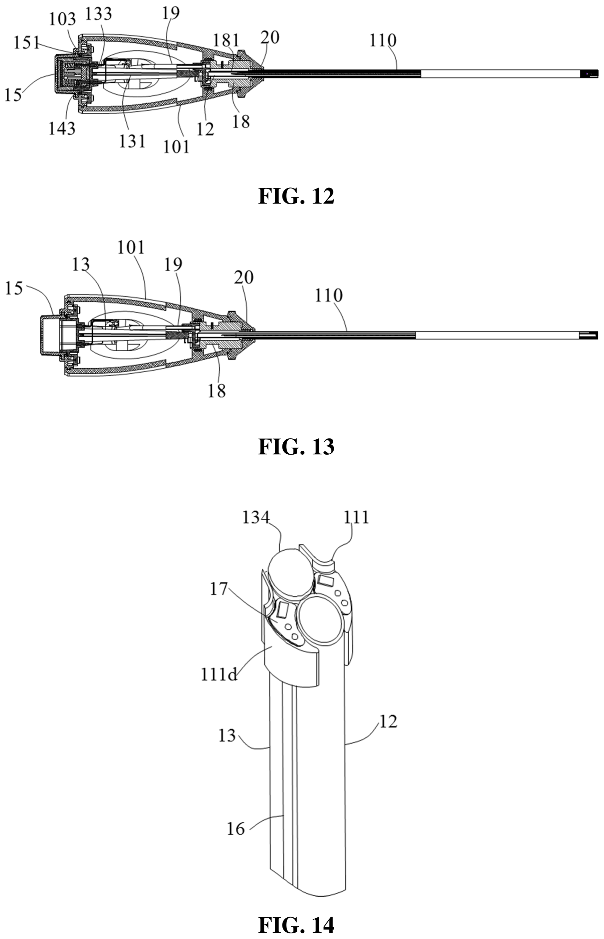

There are no limitations on the specific configuration of the insertion tube assembly 11. For example, referring to FIG. 2 and FIG. 14, the insertion tube assembly 11 includes a fixing support 111 and a glue filling layer 112. The fixing support 111 is connected to an inner wall of the insertion tube 110, and the fixing support 111 is provided with a first hole 111a and a second hole 111b. The working channel tube 12 is constrained in the first hole 111a, and the visual sleeve 13 is constrained in the second hole 111b, to allow a distal end of the working channel tube 12 and the distal end of the visual sleeve 13 to be fixed inside the insertion tube 110.

It should be noted that a shape of the first hole 111a and a shape of the second hole 111b are not limited as long as the distal end of the working channel tube 12 and the distal end of the visual sleeve 13 can be fixed inside the insertion tube 110. For example, the first hole 111a and the second hole 111b can be circular. In this way, the constraint will be more durable for the working channel tube 12 and the visual sleeve 13 with the fixing effect of the fixing support 111 improved.

The fixing support 111 can reliably fix the distal end of the working channel tube 12 and the distal end of the visual sleeve 13 inside the insertion tube 110, is able to realize the positioning of the visual sleeve 13 and the working channel tube 12 simply and effectively, and also realize the support of the distal end of the visual sleeve 13 and the distal end of the working channel tube 12. In this way, the visual sleeve 13 and the working channel tube 12 are prevented from forming a cantilever support structure in the insertion tube 110, the working channel tube 12 will not interface with the visual sleeve 13, it will reduce the shaking of the visual sleeve 13 and the working channel tube 12 when there is swing of the endoscope 1 in use, which will increase the working stability of the endoscope 1.

In some embodiments, referring to FIG. 16, a portion of an outer circumferential surface of the fixing support 111 is recessed toward a direction away from the inner wall of the insertion tube 110 to form a recessed region. The recessed region and the inner wall of the insertion tube 110 opposite to the recessed region define the liquid outlet 110a in communication with the free space.

The external fluid can flow from the liquid outlet 110a to the human body or the animal body through the liquid inlet channel and the free space, to allow the free space in the insertion tube 110 to be reasonably used without increasing the outer diameter of the insertion tube 110, and allow an end diameter of the endoscope 1 to be smaller.

There may be one liquid outlet 110a or a plurality of liquid outlets 110a. For example, there are two liquid outlets 110a. In this way, a speed can be increased at which the external fluid flows to the human body or the animal body, also it improves the operation efficiency of the endoscope 1.

There are no limitations on the specific configuration of the fixing support 111. In some embodiments, referring to FIG. 14 to FIG. 18, the fixing support 111 includes two mounting blocks 1111 separated from each other. The working channel tube 12 and the visual sleeve 13 are arranged along a first direction, and the two mounting blocks 1111 are disposed opposite to each other along a second direction and define the first hole 111a and the second hole 111b. The first direction is perpendicular to the second direction. The configuration of the two mounting blocks 1111 facilitates clamping of the working channel tube 12 and the visual sleeve 13 from two sides of the working channel tube 12 and the visual sleeve 13, ensures that the fixing support 111 can be effectively in contact with the working channel tube 12 and the visual sleeve 13, and improves the positioning reliability of the fixing support 111.

It should be noted that the first direction and the second direction may be any direction as long as the first direction is perpendicular to the second direction. Specifically, the first direction refers to a direction of a line connecting a center of the working channel tube 12 and a center of the visual sleeve 13.

When the assembly is performed, the working channel tube 12, the visual sleeve 13, and the mounting blocks 1111 may be connected together by gluing to form a pre-assembled component, which may then be arranged in the insertion tube 110.

In some embodiments, referring to FIG. 14 to FIG. 18, an outer circumferential surface of each mounting block 1111 facing toward the inner wall of the insertion tube 110 is provided with an arc-shaped surface. The arc-shaped surface is fitted against the inner wall of the insertion tube 110. The insertion tube 110 supports each mounting block 1111, and the insertion tube 110 is more tightly connected to each mounting block 1111.

For example, each mounting block 1111 and the inner wall of the insertion tube 110 may be fixed together by gluing.

In some embodiments, referring to FIG. 2 and FIG. 15, the endoscope fitting assembly includes a cable 16, and a light emitter 17 configured to provide illumination to a field of view of the image tube 14. One end of the cable 16 is electrically connected to the light emitter 17, and another end of the cable 16 extends to the handle assembly 10.

The light emitter 17 is disposed at the distal end of the insertion tube 110, specifically, on a distal end surface of the fixing support 111.

The cable 16 is configured to transmit electric energy to the light emitter 17, and the illumination required for the field of view of the image tube 14 is provided by the light emitter 17. That is, in this embodiment, the image tube 14 does not need to be integrated with the light emitter 17, which can save a mounting dimension required for the light emitter 17 and reduce the dimension requirement for the image tube 14.

There are no limitations on the cable 16.

In some embodiments, referring to FIG. 14 to FIG. 18, the fixing support 111 is provided with a through hole 111c penetrating through the fixing support 111 along an axial direction of the insertion tube 110. The cable 16 passes through the through hole 111c and is connected to the light emitter 17. The inner wall of the insertion tube 110, an outer wall of the working channel tube 12, and an outer wall of the visual sleeve 13 form a triangular region in which the cable 16 is arranged.

It should be noted that the cable 16 and the light emitter 17 do not extend into the visual sleeve 13 and the working channel tube 12, and do not affect the operation of the image tube 14 and the surgical instrument. The cable 16 makes full use of the free space in the insertion tube 110, to allow an overall structure of the endoscope fitting assembly to be more compact.

There are no limitations on the specific configuration of the light emitter 17.

In some embodiments, referring to FIG. 17, the light emitter 17 includes a circuit board 171, and a light emitting chip 172 integrated on the circuit board 171. The circuit board 171 is attached to the distal end surface of the fixing support 111.

There are no limitations on the type of the light emitting chip 172, and any component configured to emit the light can be used as the light emitting chip 172, such as a light emitting diode and the like. For example, the light-emitting chip 172 is a light emitting diode with a small size, a long lifespan, high brightness, and low heat.

There are no limitations on the number of the light emitting chip 172, and there may be one light emitting chip 172 or a plurality of the light-emitting chips 172. For example, the plurality of the light-emitting chips 172 may be uniformly arranged around the image tube 14 to form the effect of a shadowless lamp, which will not cause a visual blind area. Therefore, it can be avoided that wrinkles on an inner wall of an organ cause the shadows which affect diagnosis or surgical operation.

The circuit board 171 may be a flexible circuit board, FPC, 171. The light emitting chip 172 may be mounted on the flexible circuit board 171 by a surface assembly technology, and then the flexible circuit board 171 is mounted on the distal end surface of the fixing support 111. Meanwhile, the flexible circuit board 171 is connected to the cable 16, and the cable 16 can be connected to an external interface to realize power supply to the light emitter 17. For example, in some embodiments, the cable 16 is electrically connected to the data exchange interface 1a, and the light emitter 17 obtains the electric energy through the data exchange interface 1a to realize illumination. That is, the data exchange interface 1a may be an integrated interface of data and power of the endoscope 1, and is configured to provide power access to an electrical device in the endoscope 1.

It can be understood that the light emitter 17 is disposed insulated from an external space. For example, the light emitter 17 is disposed insulated from the free space in the insertion tube 110, to prevent the human body or the animal body from being electrocuted.

A surface of the circuit board 171 and a surface of the light emitting chip 172 are coated with the glue filling layer 112. The glue filling layer 112 can electrically insulate the circuit board 171 and an electronic device of the circuit board 171 from the environment inside the human body or the animal body, to prevent the human body or the animal body from being electrocuted. In addition, the circuit board 171 and the light emitting chip 172 are not in directly contact with the liquid inside the human body or the animal body, thereby preventing the lifespan and performance of a device such as a light emitting component from being affected.

There are no limitations on the specific configuration of the glue filling layer 112. For example, the glue filling layer 112 is formed by filling glue into a region defined by the distal end surface of the fixing support 111, and the inner wall of the insertion tube 110 and an outer wall of the optical window. An outer surface of the glue filling layer 112 forms at least a portion of the distal end surface of the insertion tube 110.

On the one hand, the glue filling layer 112 fixes the distal end surfaces of the insertion tube 110, the fixing support 111, the working channel tube 12 and the visual sleeve 13 together. On the other hand, the glue filling layer 112 plays a filling role, to allow the region between the inner wall of the insertion tube 110, the distal end surface of the fixing support 111, and the outer wall of the optical window to be filled. The outer surface of the glue filling layer 112 forms at least a portion of the distal end surface of the insertion tube 110, to allow the distal end surface of the working channel tube 12, the distal end surface of the visual sleeve 13, and a distal end surface of the insertion tube assembly 10 to be flush with each other. An end of the visual sleeve 13 and an end of the working channel tube 12 do not protrude out of the insertion tube 110, to prevent a sharp portion of the distal end of the visual sleeve 13 and a sharp portion of the distal end of the working channel tube 12 from touching or even injuring the tissue inside the human body or the animal body.

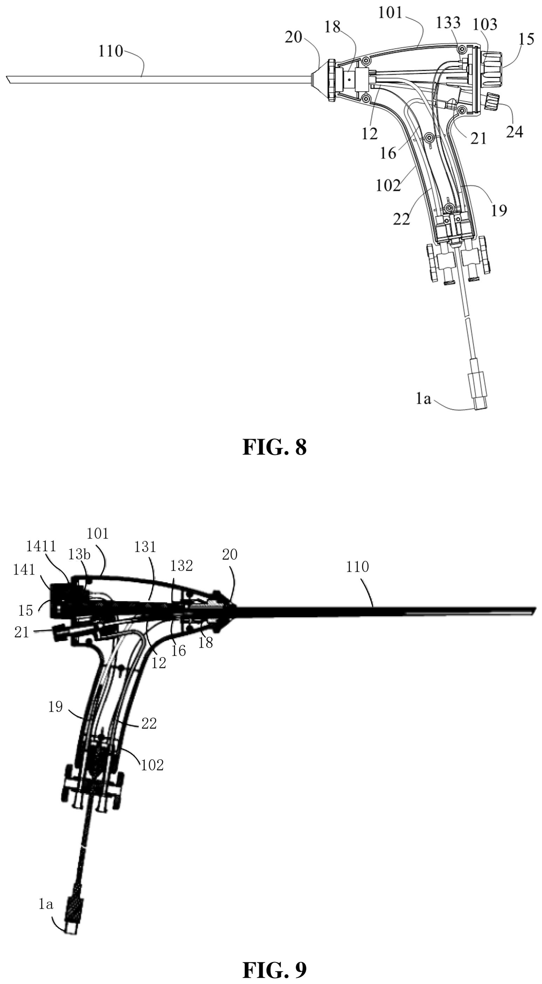

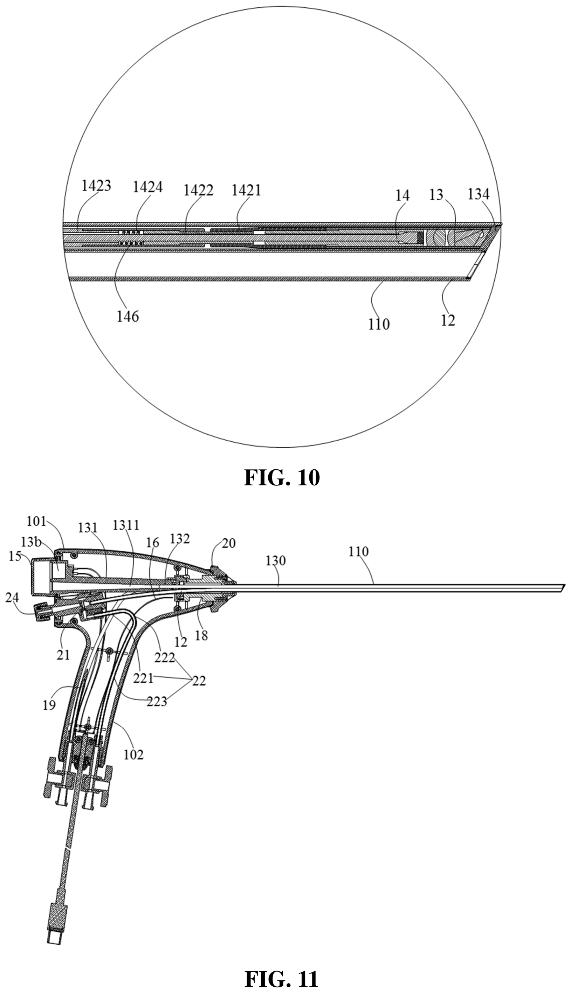

There are no limitations on the forming manner of the visual sleeve 13. The visual sleeve 13 may be of an integrally formed structure or a separately formed structure. For example, referring to FIG. 9 and FIG. 10, the visual sleeve 13 includes a sheath and the optical window. A distal end of the sheath is open, and the optical window closes a distal end portion of the sheath. The glue filling layer 112 is arranged between a circumference of the optical window and the inner wall of the insertion tube 110.

It can be understood that the arrangement of the optical window allows the distal end of the visual sleeve 13 to be in a transparent closed state, to prevent the image tube 14 from being in contact with the tissue inside the human body or the animal body, and prevent an increase in disinfection cost. In addition, the head end section 142a of the image tube 14 is movable toward the main insertion section 142b under the action of the external force, and the resilient force of the elastic member 146 actuates the head end section 142a producing the moving tendency which causes the head end section 142a to be away from the main insertion section 142b, which also provides drive for the axial alignment of the head end section 142a and the optical window. The circumference of the head end section 142a is confined by the inner circumferential surface of the visual channel 130, to allow a distal end portion of the image tube 14 to be reliably kept against and be appropriately fitted against the inner surface of the optical window. In this way, the image tube 14 can reliably acquire the image inside the human body or the animal body, and the image tube 14 can be kept against the inner surface of the optical window without other auxiliary measures to acquire the image of the tissue inside the human body or the animal body. Then the image tube 14 can be directly removed from the visual channel 130 without causing damage to the image tube 14 itself.

It can be understood that the glue filling layer 112 is arranged between the circumference of the optical window and the inner wall of the insertion tube 110. That is, the glue filling layer 112 does not affect the observation field of view and does not hinder the acquisition of the image by the image tube 14.

The glue filling layer 112 is configured to bond and fix the optical window, the fixing bracket 111 and the insertion tube 110 together to form a whole, to allow the stability of the distal end of the insertion tube 110 to be stronger. In addition, the glue filling layer 112 can also prevent the optical window from falling off and prevent tissue fluid inside the human body or the animal body from penetrating into the sheath, which affects the normal operation of the image tube 14.

Hereinafter, there are the brief descriptions on an assembly process of the insertion tube assembly 11, the working channel tube 12 and the visual sleeve 13 with reference to FIG. 2, and FIG. 9 to FIG. 18.

The working channel tube 12 and the visual sleeve 13 are mounted into the insertion tube 110 respectively, and the glue is applied to a circumferential side wall of each of two mounting blocks 1111 fitted against the inner wall of the insertion tube 110, the outer wall of the visual sleeve 13, and the outer wall of the working channel tube 12. Then the two glue-coated mounting blocks 1111 are mounted into the insertion tube 110 respectively to allow the working channel tube 12 and the visual sleeve 13 to be fixed, the insertion tube 110, the working channel tube 12 and the visual sleeve 13 can be positioned by the assisting of a jig, and an insertion depth of the fixing support 111 can also be determined by the jig. Specifically, the jig is disposed in the insertion tube 110, and after the insertion depth of the fixing support 111 determined and the glue is cured, the jig can be taken out from an end of the insertion tube 110 close to the handle assembly 10.

The light emitter 17 is arranged on the distal end surface of the fixing support 111, specifically, the light emitter 17 can be fixed to the distal end surface of the fixed bracket 111 by gluing. The light emitter 17 is connected to the cable 16, the light emitter 17 and a connection part of the light emitter 17 and the cable 16 are insulated from the outside, and the cable 16 extends out of the through hole 111c on the fixing support 111 into the handle assembly 10. A distal end of the cable 16 can be fixed at the through hole 111c by gluing, which allows the possibility of subsequent movement of the cable 16 to be reduced, and allows the possibility of fluid in the free space in the insertion tube 110 entering into the fixing support 111 to be reduced. After completing the assembly of the optical window located at the distal end portion of the visual sleeve 13, the glue filling layer 112 can be formed by filling the glue into the region defined by the distal end surface of the fixing support 111, the inner wall of the insertion tube 110 and the outer wall of the optical window. In this way, the light emitter 17 is further fixed to the distal end surface of the fixing support 111, and the distal end of the visual sleeve 13, the distal end of the working channel tube 12, and the distal end of the insertion tube 110 are further sealed and fixed.

It can be understood that before filling the glue, a special jig is inserted into the distal end of the working channel tube 12 to close the outlet 12a, to prevent the glue from entering into the working channel tube 12. After the glue filling layer 112 is cured, the special jig can be taken out.

There are no limitations on the specific configuration of the sheath of the visual sleeve 13.

In some embodiments, referring to FIG. 7 to FIG. 10, the sheath includes a first visual sleeve 131 and a second visual sleeve 132 connected to each other along a lengthwise direction of the sheath. A proximal end of the first visual sleeve 131 is provided with the visual insertion-extraction port 13a on the handle assembly 10. The first visual sleeve 131 is provided with a first visual channel 1311, a proximal end of the second visual sleeve 132 extends into and is fixed in the first visual channel 1311, and another end of the second visual sleeve 132 is disposed in the insertion tube 110.

The image tube 14 passes through the first visual sleeve 131 through the visual insertion-extraction port 13a and then passes through the second visual sleeve 132 into the insertion tube 110. That is, the image tube 14 is never in contact with the insertion tube 110. A cross-section of the first visual channel 1311 gradually decreases in a direction from the proximal end to the distal end, which plays a guide and positioning role, facilitates the insertion of the image tube 14, and also allows the second visual sleeve 132 to be better fixed in the first visual channel 1311.

There are no limitations on the connection manner of the insertion tube 110 and the handle assembly 10. The insertion tube 110 and the handle assembly 10 may be directly connected to each other, or connected to each other by other components. In some embodiments, referring to FIG. 7 to FIG. 10, the endoscope fitting assembly includes a joint 18, and the handle assembly 10 is provided with a mounting port 10a. The joint 18 is rotatably arranged in the mounting port 10a, and the proximal end of the insertion tube 110 is hermetically inserted into and fixed in the joint 18. In this way, the joint 18 can drive the insertion tube 110 to rotate, to allow an orientation of the insertion tube 110 to be adjusted.

There are no limitations on the specific configuration of the joint 18. In some embodiments, referring to FIG. 7 to FIG. 10, the joint 18 is provided with a flow channel 181 into which an end of the insertion tube 110 extends. The endoscope fitting assembly includes a liquid inlet tube 19, one end of the liquid inlet tube 19 is configured to introduce the external fluid, and another end of the liquid inlet tube 19 is connected to an inlet of the flow channel 181. That is, the flow channel 181 is in communication with a free space between a tail end of the liquid inlet tube 19 and the proximal end of the insertion tube 110. The flow channel 181 and a space in the liquid inlet tube 19 define at least a portion of the liquid inlet channel.

The external fluid flows into the flow channel 181 through the liquid inlet tube 19, and then flows from the liquid outlet 110a into the human body or the animal body through the free space of the insertion tube 110. In this way, the external fluid flows into the human body or the animal body using the unoccupied free space in the insertion tube 110, to allow the observed region to be filled with the liquid and inflated, for example, to allow the uterine cavity to be filled with water, which facilitates the better observation by the image tube 14 without additionally providing a liquid inlet tube in the insertion tube 110. In this way, it's probable to reduce the outer diameter of the insertion tube 110, there is a smaller aperture diameter of the natural lumen or minimal invasive incision required, to improve the diagnosis and treatment efficiency of the endoscope 1, and reduce damage to the patient or the animal body.

There are no limitations on the manner in which the liquid inlet tube 19 introduces the external fluid. For example, as shown in FIG. 2, the liquid inlet tube 19 is provided with a water inlet valve 23, and the introduction of the external fluid into the human body or the animal body can be started or stopped according to the actual use needs.

In some embodiments, referring to FIG. 2 and FIG. 7, the handle assembly 10 includes a handle 101, and a handgrip 102 disposed at a bottom side of the handle 101. The working channel tube 12 and the visual sleeve 13 extend along a lengthwise direction of the handle 101. The mounting port 10a is disposed at an end of the handle 101 close to the insertion tube 110, at least a portion of the joint 18 is disposed inside a distal end of the handle 101. In this way, the joint 18 can drive the insertion tube 110 to rotate, to allow the orientation of the insertion tube 110 to be adjusted, and the joint 18 does not scratch against components of the insertion tube 110.

An end of the liquid inlet tube 19 away from the joint 18 extends along the handgrip 102 to an end of the handgrip 102 away from the handle 101, which can avoid interference to the operator during use.

In some embodiments, referring to FIG. 2 and FIG. 7, the endoscope fitting assembly includes a turntable 20 disposed outside the handle assembly 10. The turntable 20 is arranged to cover around an outer peripheral side of the insertion tube 110, and the turntable 20 is rotatably arranged to case around the joint 18 and detachably fixed to the joint 18.

That is, the turntable 20 can drive the joint 18 to rotate, thereby driving the insertion tube 110 to rotate. When in use, the operator can drive the insertion tube 110 and components such as the visual sleeve 13 and the image tube 14 disposed in the insertion tube 110 to rotate by rotating the turntable 20, which can satisfy the adjustment requirement for the orientation of the endoscope 1 and the adjustment requirement for the field of view of the surgery.

There are no limitations on the connection manner of the joint 18 and the turntable 20. For example, the joint 18 and the turntable 20 may be fixed to each other by a screw.

In some embodiments, referring to FIG. 2, FIG. 7, and FIG. 22, the endoscope fitting assembly includes a coupling part 21 and a liquid discharging tube 22.

The coupling part 21 is fixed to the handle assembly 10. There are no limitations on the fixing manner of the coupling part 21 and the handle assembly 10. For example, referring to FIG. 2 to FIG. 7, the proximal end portion of the handle assembly 10 is provided with a through hole 10b, and the coupling part 21 passes through the through hole 10b and is fixed to the proximal end of the handle assembly 10.

Referring to FIG. 2 and FIG. 22, the coupling part 21 includes a first coupling sleeve 211 and a second coupling sleeve 212 butted with each other. A distal end of the first coupling sleeve 211 is provided with an insertion cavity 211a, and a proximal end of the second coupling sleeve 212 is inserted into the insertion cavity 211a. A cavity region 2111 is formed between a portion of a circumferential surface of the second coupling sleeve 212 and an inner wall of the insertion cavity 211a. A side wall of the insertion cavity 211a corresponding to the cavity region 2111 is provided with a coupling interface 211b.

A proximal end of the working channel tube 12 extends into and is fixed inside a distal end of the second coupling sleeve 212, and is in communication with the cavity region 2111. In this way, the surgical instrument can extend into the working channel tube 12 through the cavity region 2111 to perform the surgical operation. The extension of the surgical instrument does not interfere with other components, which facilitates the operation.

There are no limitations on the fitting manner of the working channel tube 12 and the second coupling sleeve 212. The second coupling sleeve 212 is provided with a second axial hole 212a, and the proximal end of the working channel tube 12 is fixed inside the second axial hole 212a. There are no limitations on the fixing manner of the working channel tube 12 and the second axial hole 212a, for example to be intended to include, but is not limited to, gluing.

An end of the liquid discharging tube 22 is in communication with the coupling interface 211b, to discharge fluid from the working channel tube 12.

It can be understood that in order to meet the requirements of lesion observation or surgery, the diameter of the insertion tube 110 should be as small as possible, to allow damage to the human body or the animal body to be reduced.

In the embodiment of the present application, the liquid discharging tube 22 is in communication with the working channel tube 12 through the coupling part 21, and the liquid discharging tube 22 is not disposed inside the insertion tube 110. The liquid inside the human body or the animal body can be discharged using the working channel tube 12 without additionally providing a separate liquid discharging tube in the insertion tube 110. In this way, the space of the insertion tube 110 is not occupied, there is the smaller aperture diameter of the natural lumen or the minimal invasive incision required, to increase the working reliability of the endoscope 1.

Specifically, when the insertion tube 110 is invasive in the human body or the animal body through the natural lumen of the human body or the animal body, the smaller diameter of the insertion tube 110 allows the use of an auxiliary expanding tool to be reduced, and allows damage to the lumen of the human body or the animal body to be effectively reduced. When the insertion tube 110 is invasive in the human body or the animal body from the minimally invasive incision, the smaller diameter of the insertion tube 110 allows a size of the stoma to be effectively reduced, allows damage to the patient or the animal to be reduced, and allows the rapid healing of the patient or the animal to be realized.

There are no limitations on the manner in which the liquid discharging tube 22 discharges the liquid inside the human body or the animal body. For example, a negative pressure device may be externally connected to an end of the liquid discharging tube 22 away from the working channel tube 12. When suction is needed, the negative pressure device generates a negative pressure, and further the negative pressure occurs in the working channel tube 12, which facilitate the suction of the liquid to be sucked out.

Specifically, in the embodiment of the present application, if it is necessary to suck and discharge the liquid during surgery, the negative pressure device is externally connected to the liquid discharging tube 22 to generate the negative pressure. The liquid from the human body or the animal body is diverted in the coupling part 21 after passing through the working channel tube 12, flows out from the coupling interface 211b, and then flows out from the liquid discharging tube 22.

Referring to FIG. 22, a space expanding section 2121 that expands toward the first coupling sleeve 211 is formed inside the proximal end of the second coupling sleeve 212.

The surgical instrument can enter into the working channel tube 12 through the cavity region 2111 and the space expanding section 2121 to perform the surgical operation.

It should be noted that when the surgery or examination is performed using the endoscope 1, if it is necessary to suck and discharge the liquid while operating the surgical instrument, a portion of the liquid from the human body or the animal body may unexpectedly leak (flow out or spill) along the working channel tube, instead of completely controllable being discharged through the liquid discharging tube. In addition, unexpected liquid leakage from the working channel tube may accidentally transfer pathogens to the operator or contaminate the surgical environment, and may even cause a risk of infection to the patient or the animal body.

Therefore, in the embodiment of the present application, the space expanding section 2121 allows an effective volume of the flow channel to be increased. When the liquid from the human body or the animal body flows through the space expanding section 2121, a flow velocity decreases and a static pressure increases. When the liquid is sucked and discharged, the negative pressure generated by the external negative pressure device guides the liquid from the human body or the animal body with the increased static pressure to tend to flow toward the coupling interface 211b along the space expanding section 2121, and then to flow out from the liquid discharging tube 22.

In the endoscope fitting assembly according to the embodiment of the present application, the liquid discharging tube 22 is disposed outside the insertion tube 110, and the liquid in the human body or animal body is diverted in the coupling part 21 after passing through the working channel tube 12, to flow out from the coupling interface 211b and then flow out from the liquid discharging tube 22. In this way, it is not necessary to additionally provide a separate liquid discharging tube in the insertion tube 110, there is no occupation of the space of the insertion tube 110, it's unnecessary to enlarge the outer diameter of the insertion tube 110, and there is the smaller aperture diameter of the natural lumen or the minimal invasive incision required. The space expanding section 2121 in the coupling part 21 is in communication with the cavity region 2111 and the insertion cavity 211a, to allow the effective volume of the flow channel to be increased rapidly. When the liquid from the human body or the animal body flows through the space expanding section 2121, the flow velocity decreases and the static pressure increases, to allow the liquid from the human body or animal body to more concentratedly flow to the coupling interface 211b. In this way, it's probable to reduce the liquid from the human body or animal body leaking from a proximal end portion of the cavity region 2111. Furthermore, the space expanding section 2121 allows an insertion space of the surgical instrument to be expanded, such that it's probable to reduce the surgical instrument being confined in a dead angle.

In some embodiments, referring to FIG. 22, a cross-sectional area of the space expanding section 2121 increases gradually in a direction away from the working channel tube 12.

The flow velocity of the fluid is affected by a flow rate and a cross-section area of flow channel. When the flow rate of the fluid remains constant, the cross-section area of flow channel increases and the flow velocity of the fluid decreases. The cross-sectional area of the space expanding section 2121 increases gradually, that is, the cross-section area of flow channel for the fluid increases, the flow velocity of the fluid decreases, and the flow stability of the fluid is enhanced, which facilitates the space expanding section 2121 to guide the fluid to concentratedly flow to the coupling interface 211b.

Specifically, when the liquid is discharged and when the liquid from the human body or the animal body flows to the space expanding section 2121 through the working channel tube 12, the space expanding section 2121 makes the cross-section area of flow channel for the liquid from the human body or the animal body to be increased, so the flow velocity of liquid from the human body or the animal body will decrease and the static pressure of the liquid from the human body or the animal body will increase, such that the liquid from the human body or animal body concentratedly flows to the coupling interface 211b, and is discharged from the liquid discharging tube 22, also it's probable to reduce the liquid leaking of the human body or animal body from the proximal end portion of the cavity region 2111. The gradual increase in the cross-sectional area of the space expanding section 2121 also facilitates the insertion of the surgical instrument, and reduces the probability of the surgical instrument being confined in the dead angle.

In some embodiments, referring to FIG. 22, the second coupling sleeve 212 includes a hermitic axial section 2122, and a circumferential surface of the hermitic axial section 2122 is in hermitic interference fit with the insertion cavity 211a. It realizes a hermitic connection between the first coupling sleeve 211 and the second coupling sleeve 212 such that the fluid does not leak out of the second coupling sleeve 212.

It can be understood that the circumferential surface of the hermitic axial section 2122 is coated with an adhesive, and further bonded to the insertion cavity 211a, to realize a fixed connection between the second coupling sleeve 212 and the first coupling sleeve 211. In this way, it increases the connection stability between the first coupling sleeve 211 and the second coupling sleeve 212. The liquid from the human body or the animal body does not leak out of the circumferential surface of the hermitic axial section 2122 through the cavity region 2111.

An axial end surface 2122a of the hermitic axial section 2122 is spaced apart from a cavity end surface 211c of the insertion cavity 211a facing toward the axial end surface 2122a, and the coupling interface 211b is disposed at a bottom of a circumferential side wall between the axial end surface 2122a and the cavity end surface 211c. The axial end surface 2122a is spaced apart from the cavity end surface 211c, which allows the liquid accommodation space to be expanded, such that it increases the cross-section area of flow channel for the liquid from the human body or the animal body. In this way, the liquid from the human body or the animal body can smoothly flow out of the coupling interface 211b.

In some embodiments, referring to FIG. 22, the second coupling sleeve 212 includes a dwindling axial section 2123 connected to a proximal end of the hermitic axial section 2122, and an outer diameter of the dwindling axial section 2123 is less than an outer diameter of the hermitic axial section 2122. The space expanding section 2121 extends from an interior of the hermitic axial section 2122 to a proximal end surface of the dwindling axial section 2123.

In this way, on the one hand, it increases an extension space of the space expanding section 2121, which allows the cross-section area of flow channel for the liquid from the human body or the animal body. On the other hand, it also increases the external liquid accommodation space of a side of the space expanding section 2121 facing toward the coupling interface 211b, specifically, it increases the liquid accommodation space of the cavity region 2111. In this way, the space expanding section 2121 is in communication with the cavity region 2111, and the liquid from the human body or the animal body can enter into a large space after flowing out of the space expanding section 2121. Therefore, it increases the cross-section area of flow channel for the liquid from the human body or the animal body flowing through the space expanding section 2121 and the cavity region 2111, also it increases the static pressure of the liquid from the human body or the animal body flowing through the space expanding section 2121 and the cavity region 2111. In addition, it increases a diameter of the coupling interface 211b, which facilitates the liquid from the human body or the animal body to more concentratedly flow from the coupling interface 211b to the liquid discharging tube 22.

In some embodiments, referring to FIG. 22, the first coupling sleeve 211 is provided with a first axial hole 211d, and the insertion cavity 211a is positioned at a distal end of the first axial hole 211d. An inner diameter of the insertion cavity 211a is greater than an inner diameter of the distal end of the first axial hole 211d, and the cavity end surface 211c is formed at a boundary of the insertion cavity 211a and the first axial hole 211d. The first coupling sleeve 211 is provided with an annular platform 2112 positioned in the insertion cavity 211a, the annular platform 2112 surrounds a distal end portion of the first axial hole 211d, and the annular platform 2112 is spaced apart from a circumferential side wall of the insertion cavity 211a.

It can be understood that when the surgical instrument is required to perform the surgical operation, the surgical instrument can be inserted from a proximal end of the first axial hole 211d, and enter into the working channel tube 12 through the cavity region 2111 and the space expanding section 2121 to perform the surgical operation.

On the one hand, the arrangement of the annular platform 2112 further allows a liquid accommodation space in the coupling part 21 to be expanded for the liquid from the human body or the animal body flowing out of the space expanding section 2121, specifically, further allows the liquid accommodation space of the cavity region 2111 to be expanded, which facilitates the liquid from the human body or the animal body to more concentratedly flow from the coupling interface 211b to the liquid discharging tube 22. On the other hand, the annular platform 2112 also facilitates the surgical instrument to be accurately inserted into the second axial hole 212a through the cavity region 2111 when the surgical instrument is inserted from the first axial hole 211d, which allows the surgical instrument to enter into the working channel tube 12 to perform the operation. In this way, it's probably to reduce that the surgical instrument is confined in the dead angle during insertion and cannot be inserted into the working channel tube 12 due to the large space of the cavity region 2111. Therefore, the annular platform 2112 does not affect the normal insertion of the surgical instrument while ensuring that the liquid accommodation area of the cavity region 2111 is as large as possible.

In some embodiments, referring to FIG. 22, an end surface of the annular platform 2112 is spaced apart from an end face of the dwindling axial section 2123. In this way, it facilitates the normal insertion of the surgical instrument and reduces the risk of the fluid flowing from the proximal end portion of the cavity region 2111 into the first axial hole 211d.

In some embodiments, referring to FIG. 2, FIG. 5, and FIG. 22, the endoscope fitting assembly includes a screw cap 24 detachably arranged on a proximal end of the first coupling sleeve 211. The screw cap 24 includes a cap body 241 and a bulge 242 protruding from an interior of the cap body 241 toward the first coupling sleeve 211. The bulge 242 extends into the first axial hole 211d and seals the first axial hole 211d.

When the surgical instrument is not used, the bulge 242 seals the first axial hole 211d, and the first axial hole 211d is in a sealed state. The liquid from the human body or the animal body does not flow out of the first axial hole 211d. In this way, the creation of contamination, which impacts the normal surgical operation, is avoided.

When the surgical instrument is used, the first axial hole 211d is in an open state, and if the liquid needs to be discharged, the surgical instrument can still be retained in the working channel tube 12, and the negative pressure device externally connected to the liquid discharging tube 22 generates the negative pressure. The liquid from the human body or the animal body flows from the coupling interface 211b to the liquid discharging tube 22 through the working channel tube 12, the space expanding section 2121, and the cavity area 2111, without flowing out of the first axial hole 211d to cause the contamination. In this way, it realizes the purpose of discharging the liquid while using the surgical instrument and increases the working efficiency of the endoscope 1.

The material of the bulge 242 is not limited as long as the bulge 242 can seal the first axial hole 211d. For example, the bulge 242 may be made of medical plastic. In other embodiments, each of the bulge 242 and the cap body 241 may be made of medical plastic, and the bulge 242 and the cap body 241 may be integrally formed. That is, the screw cap 24 may be formed as an integrally formed structure.

In some embodiments, referring to FIG. 22, a cross-sectional area of the first axial hole 211d decreases continuously in a direction from the proximal end to the distal end of the first axial hole 211d.

In the process of the bulge 242 extending into the first axial hole 211d, the cross-sectional area of the first axial hole 211d decreases continuously, to allow the bulge 242 to be fixed in the first axial hole 211d. In this way, it realizes the sealing of the first axial hole 211d and increases the adaptability and stability of the screw cap 24 and the first coupling sleeve 211.

In addition, a slow dwindling structure of the first axial hole 211d also facilitates insertion of the surgical instrument and reduces the probability of insertion of the surgical instrument into the dead angle. An opening of the proximal end of the first axial hole 211d is large, which facilitates insertion of the surgical instrument. An opening of the distal end of the first axial hole 211d which is small, faces toward an opening of a proximal end of the space expanding section 2121 which is large. In this way, it reduces the probability of insertion of the surgical instrument into the dead angle, and the surgical instrument can smoothly enter into the opening of the proximal end of the space expanding section 2121 after extending out of the opening of the distal end of the first axial hole 211d, and then extend into the working channel tube 12 to perform the surgical operation.

It should be noted that the proximal end portion of the handle assembly 10 is provided with a circular plate, and the through hole 10b is disposed on the circular plate. When the turntable 20 rotates, the joint 18 will rotate together, which will drive the insertion tube 110 and the circular plate to rotate too. That is, when the turntable 20 rotates, the joint 18, the coupling part 21, the insertion tube 110, the visual sleeve 13, and the image tube 14 will rotate together which facilitate the orientation adjustment of the endoscope 1 and the adjustment of view field of surgery.

There are no limitations on the arrangement manner of the liquid discharging tube 22. In some embodiments, referring to FIG. 7, an end of the liquid discharging tube 22 away from the coupling part 21 extends along the handgrip 102 to an end of the handgrip 102 away from the handle 101, which can avoid interference to the operator during use.

There are no limitations on the manner in which the liquid discharging tube 22 is in communication with the coupling interface 211b. The liquid discharging tube 22 and the coupling interface 211b may be directly in communication with each other or may be in communication with each other by other components.

In some embodiments, referring to FIG. 2 and FIG. 22, the endoscope fitting assembly includes a coupling head 213. A first coupling cavity 213a and a second coupling cavity 213b in communication with each other are formed inside the coupling head 213. The first coupling cavity 213a is in communication with the coupling interface 211b, and the second coupling cavity 213b is in communication with an end of the liquid discharging tube 22 close to the handle 101. The first coupling cavity 213a is disposed along a lengthwise direction of the handgrip 102, and the second coupling cavity 213b is disposed along the lengthwise direction of the handle 101.

It can be understood that if the liquid discharging tube 22 is directly connected to the coupling interface 211b and a distance between the coupling interface 211b and an inner wall of the handle assembly 10 is small, there is insufficient space for connecting the liquid discharging tube 22 and the coupling interface 211b, which makes it inconvenient to realize the effective connection between the liquid discharging tube 22 and the coupling interface 211b. In the embodiment of the present application, the coupling head 213 is arranged to change a connection direction of the liquid discharging tube 22, which allows the liquid discharging tube 22 to be connected to the coupling head 213 at a place with a large space, thereby realizing communication between the liquid discharging tube 22 and the coupling interface 211b. In this way, it facilitates the mounting of the liquid discharging tube 22 during production and improves the connection stability of the liquid discharging tube 22.

The specific configuration of the coupling head 213 is not limited as long as the liquid discharging tube 22 and the coupling interface 211b may be in communication with each other.

In some embodiments, referring to FIG. 8, the liquid discharging tube 22 includes a first liquid discharging section 221, a bent section 222 and a second liquid discharging section 223. A portion of the liquid discharging tube 22 protrudes towards a direction away from the coupling part 21 to form the bent section 222. The first liquid discharging section 221 extends into and is fixed in the second coupling cavity 213b, and the second liquid discharging section 223 extends along the lengthwise direction of the handgrip 102. The bent section 222 connects the first liquid discharging section 221 and the second liquid discharging section 223.

In this way, a shape design of the liquid discharging tube 22 facilitates the full utilization of a space inside the handle assembly 10, facilitates the assembly of the coupling head 213 during production, the liquid discharging tube 22 and the handle assembly 10, facilitates the discharge of the liquid discharging tube 22 of the liquid from the human body or the animal body flowing out from the coupling interface 211b, and avoids backflow. In addition, the shape design of the liquid discharging tube 22 can also leave a spare space inside the handle assembly 10, which facilitates the mounting and orientation adjustment of components such as the insertion tube 110 and the like.

It can be understood that in the process of the operation of the endoscope 1, it is necessary to rotate the insertion tube 110 to allow the orientation adjustment of other components such as the insertion tube 110 and the like, which facilitates the acquisition of the high-quality image. The shape design of the liquid discharging tube 22 allows the liquid discharging tube 22 to leave sufficient redundancy inside the handle assembly 10. In this way, when the insertion tube 110 is rotated to adjust the orientation of the insertion tube 110, the redundancy of the liquid discharging tube 22 is contributed to no interference between the liquid discharging tube 22 and the rotating insertion tube 110, it increases the working reliability of the endoscope 1.

Hereinafter, the use process of the endoscope 1 will be described by taking an example that the observed area is uterine cavity.

Before the endoscope 1 is inserted into the uterine cavity, the image tube 14 is inserted into the visual sleeve 13 through the visual insertion-extraction port 13a, the distal end of the image tube 14 is kept against the optical window, the head end section 142a of the image tube 14 axially moves toward the main insertion section 142b under the action of the external force from the inner surface of the optical window to the main insertion section 142b. The elastic member 146 is compressed and deformed, the resilient force of the elastic member 146 actuates the head end section 142a producing the moving tendency which causes the head end section 142a to axially be away from the main insertion section 142b, which also provides drive for the axial alignment of the head end section 142a and the optical window. The circumference of the head end section 142a is confined by the inner circumferential surface of the visual channel 130, to allow the distal end portion of the image tube 14 to be reliably kept against and be appropriately fitted against the distal end portion of the visual sleeve 13. The second snapping part 151 on the end cap 15 is snapped to the first snapping part 103 on the image tube 14. A water inlet end of the liquid inlet tube 19 is connected to a hysteroscopy pump, and the hysteroscopy pump is configured to provide uterine distention liquid. The uterine distention liquid flows into the uterine cavity through the liquid inlet tube 19, the flow channel 181, the free space in the insertion tube 110, and the liquid outlet 110a, to allow the uterine cavity to be filled with the liquid and inflated. The data exchange interface 1a is connected to the external device, the cable 16 is electrically connected to the data exchange interface 1a, and the light emitter 17 acquires the power required for illumination from the data exchange interface 1a through the cable 16.

The distal end of the endoscope 1 is placed into the uterine cavity, and the illuminator 17 provides illumination to the image tube 14. The uterine distention liquid is injected through a certain pressure and speed, to allow a uterine cavity to be filled and the field of view to be clear. An interior of the uterine cavity is observed through the image tube 14. The photoelectric conversion component 144 converts the optical signal into the electrical signal, and the electrical signal is transmitted to the plurality of first electrical terminals 143, the plurality of second electrical terminals 133 and the data exchange interface 1a through the signal line 147. The external device converts the image electrical signal into a video image and displays the video image. If a further surgical operation is needed, the corresponding surgical instrument can be inserted into the uterine cavity from the working channel tube 12 through a second opening 21b and a first opening 21a to perform the surgical operation.

In surgery, if suction is needed, the negative pressure device can be connected to perform the suction. At this time, the surgical instrument can still be retained in the working channel tube 12, or the operator can perform the surgical operation while the suction is performed. When it is necessary to suck and discharge the liquid, the negative pressure device is externally connected to the liquid discharging tube 22 to generate the negative pressure, to allow the liquid inside the uterine cavity to be discharged from the liquid discharging tube 22 through the working channel tube 12. The surgical instrument is taken out after the surgery is completed. Finally, the end cap 15 is taken out, the image tube 14 is extracted out from the visual sleeve 13, and the image tube 14 is simply disinfected and wiped for next use. The insertion tube assembly 11, the working channel tube 12, the visual sleeve 13 and the like can be discarded as medical disposables.

In the description of the present application, the description of the terms "one embodiment", "some embodiments", "examples", "specific examples", "some examples" and the like means that specific features, structures, materials, or characteristics described in combination with the embodiment or example are included in at least one embodiment or example of embodiments of the present application. In the present application, the schematic expressions of the above terms should not be necessarily referred to the same embodiments or examples. In addition, the specific features, the structures, the materials, or the characteristics described may be combined in any one or more embodiments or examples in a suitable manner. Furthermore, different embodiments or examples and features of different embodiments or examples described in the present application may be combined by those skilled in the art without contradicting each other.

What described above are merely preferable embodiments of the present application, and are not intended to limit the present application. Various changes and variations can be made in the present application for those skilled in the art. Any modification, equivalent replacement, improvement and the like made within the spirit and principle of the present application should be included in the scope of protection of the present application.

## Claims

1. An image tube configured to be in insertion-extraction fit with an endoscope fitting assembly, comprising:
a core tube comprising a main insertion section and a head end section in a lengthwise direction of the core tube, a proximal end of the head end section being connected to a distal end of the main insertion section, the head end section and/or the main insertion section being axially movable close to each other under an action of an external force; and
an elastic member disposed in the core tube, the elastic member being compressible and resilient, to actuate the head end section producing moving tendency which causes the head end section to axially be away from the main insertion section.

2. The image tube according to claim **1,** wherein the head end section comprises a first body tube and a first casing tube, and the first casing tube is provided as a separate component and fixed to a proximal end of the first body tube; and
the main insertion section comprises a second body tube and a second casing tube, the second casing tube is provided as a separate component and fixed to a distal end of the second body tube, and the head end section and the main insertion section are connected to each other through the first casing tube and the second casing tube.

3. The image tube according to claim 2, wherein a first dwindling section is formed at the distal end of the second body tube, the first dwindling section is inserted inside a proximal end of the second casing tube, the first casing tube is arranged inside a distal end of the second casing tube, one end of the elastic member is kept against an end of the second body tube, and another end of the elastic member is kept against an end of the first casing tube.

4. The image tube according to claim 3, wherein the second casing tube is provided with an accommodating lumen in which the first dwindling section is accommodated, the first casing tube comprises a first casing section and a second casing section in a lengthwise direction of the first casing tube, an outer diameter of the first casing section is greater than an outer diameter of the second casing section, the first casing section is inserted into the accommodating lumen, a distal side wall of the accommodating lumen protrudes inwardly to form a limiting wall, a proximal end portion of the first casing section is kept against the elastic member, and a distal end portion of the first casing section detachably abuts against the limiting wall.

5. The image tube according to claim 4, wherein a circumferential outer side wall of the first casing section is tangent to a side wall of the accommodating lumen.

6. The image tube according to claim 2, wherein a second dwindling section is formed at a proximal end of the second casing tube, the second dwindling section is inserted inside the distal end of the second body tube, the first casing tube is arranged around a distal end of the second casing tube, one end of the elastic member is kept against a distal end portion of the second body tube, and another end of the elastic member is kept against a proximal end portion of the first casing tube.

7. The image tube according to claim 2, wherein the second casing tube is arranged around the first casing tube, and a distal end portion of the second casing tube is axially spaced apart from a proximal end portion of the first body tube to define a movement stroke of the head end section in an axial direction; or the first casing tube is arranged around the second casing tube, and a proximal end portion of the first casing tube is axially spaced apart from a distal end portion of the second body tube to define a movement stroke of the head end section in an axial direction.

8. The image tube according to claim 1, wherein the elastic member is a compression spring.

9. The image tube according to claim 1, wherein a distal end of the head end section is integrated with an optical component, and a photoelectric conversion component for converting an optical signal of the optical component into an electrical signal, the image tube comprises a signal line disposed in the core tube and extending along the lengthwise direction of the core tube, and a distal end of the signal line is connected to the photoelectric conversion component.

10. The image tube according to claim 9, wherein the image tube comprises a disc part connected to a proximal end of the core tube, the disc part comprises a disc body and a protrusion protruding in a direction from the disc body to the head end section, a distal end of the protrusion is provided with a plurality of first electrical terminals, and a proximal end of the signal line is electrically connected to the plurality of first electrical terminals.

11. An endoscope, comprising:
an endoscope fitting assembly and the image tube according to any one of claims 1 to 10;
the endoscope fitting assembly comprising:
a handle assembly;
an insertion tube assembly, a proximal end of the insertion tube assembly being connected to the handle assembly, the insertion tube assembly comprising an insertion tube, and a distal end surface of the insertion tube being provided with a liquid outlet; and
a visual sleeve disposed inside the insertion tube, the visual sleeve being provided with a visual channel, and a distal end portion of the visual sleeve being provided with a closed optical window;
wherein the core tube is insertable into the visual channel, and a distal end portion of the head end section is configured to be kept against an inner surface of the optical window.

12. The endoscope according to claim 11, wherein the visual sleeve is provided with a visual insertion-extraction port at a proximal end of the handle assembly, and the image tube is in detachable insertion-extraction fit with the visual channel through the visual insertion-extraction port.

13. The endoscope according to claim 12, wherein the visual sleeve comprises a first visual sleeve and a second visual sleeve connected to each other along a lengthwise direction of the visual sleeve, a proximal end of the first visual sleeve is provided with the visual insertion-extraction port at the proximal end of the handle assembly, the first visual sleeve is provided with a first visual channel, a proximal end of the second visual sleeve extends into and is fixed in the first visual channel, and another end of the second visual sleeve is disposed in the insertion tube, and a cross-section of the first visual channel gradually decreases in a direction from a proximal end to a distal end.

14. The endoscope according to claim 12, wherein the visual sleeve is provided with a plurality of second electrical terminals, the image tube comprises a disc part, the disc part comprises a disc body, and a protrusion protruding from the disc body toward the visual sleeve, a side of the protrusion facing toward the visual sleeve is integrated with a plurality of first electrical terminals, each of the plurality of first electrical terminals is detachably connected to and conductively connected to a respective one of the plurality of second electrical terminals, the endoscope fitting assembly comprises a data exchange interface configured to interact with an external device, and the data exchange interface is in electrical communication with the plurality of second electrical terminals.

15. The endoscope according to claim 14, wherein a proximal end of the visual sleeve extends out of a proximal end portion of the handle assembly, a proximal end portion of the visual sleeve is provided with a recess and the visual insertion-extraction port, the proximal end portion of the visual sleeve is recessed toward a direction close to a distal end of the insertion tube to form the recess, the plurality of second electrical terminals are disposed at a distal end of the recess and penetrate through a groove wall of the recess along an insertion-extraction direction of the image tube, one end of each second electrical terminal is exposed in the recess, another end of each second electrical terminal extends into a space in the handle assembly, and the protrusion extends into and is fixed in the recess.

16. The endoscope according to claim 11, wherein the endoscope fitting assembly comprises a cable, and a light emitter configured to provide illumination to a field of view of the image tube, the light emitter is disposed at a distal end of the insertion tube, one end of the cable is electrically connected to the light emitter, and another end of the cable extends to the handle assembly.

17. The endoscope according to claim 16, wherein the endoscope fitting assembly comprises a data exchange interface configured to interact with an external device, and the cable is electrically connected to the data exchange interface.

18. The endoscope according to claim 11, wherein the optical window comprises an incident lens element, and a reflecting prism disposed in the visual sleeve, the incident lens element is disposed at an end of the visual sleeve and located on a light incident side of the reflecting prism, the incident lens element is configured to receive external light, and refract and converge the external light to the reflecting prism, and the reflecting prism is configured to transmit light to a light incident portion of the image tube in the visual channel by reflection.

19. The endoscope according to claim 18, wherein a light emitting surface of the reflecting prism is perpendicular to a lengthwise direction of the visual sleeve.

20. The endoscope according to claim 18, wherein a distal end surface of the incident lens element is an inclined surface, an arc-shaped groove is formed at a side of the incident lens element facing toward the reflecting prism, a surface of the arc-shaped groove is a light emitting surface of the incident lens element, and a light incident surface of the reflecting prism covers the arc-shaped groove.

21. The endoscope according to claim 20, wherein the reflecting prism is provided with at least a first reflecting surface and a second reflecting surface disposed face to face, the first reflecting surface is inclined toward the arc-shaped groove, and the second reflecting surface is located on a light emitting side of the first reflecting surface, to allow light emitted from the arc-shaped groove to be emitted after being reflected by the first reflecting surface and the second reflecting surface in sequence, or to be emitted after being subjected to one or more light processing.

22. The endoscope according to claim 11, wherein the handle assembly is provided with a liquid inlet channel, an interior of the insertion tube is provided with a free space in communication with the liquid inlet channel and the liquid outlet, the endoscope fitting assembly comprises a working channel tube, a working channel through which a surgical instrument passes is formed inside the working channel tube, the working channel tube is arranged inside the insertion tube and is provided with an outlet at the distal end surface of the insertion tube, wherein the free space is an unoccupied space in the insertion tube.

23. The endoscope according to claim 22, wherein the endoscope fitting assembly comprises a joint, the handle assembly is provided with a mounting port, the joint is rotatably arranged in the mounting port, and a proximal end of the insertion tube is hermetically inserted into and fixed in the joint.

24. The endoscope according to claim 23, wherein the joint is provided with a flow channel into which an end of the insertion tube extends, the endoscope fitting assembly comprises a liquid inlet tube, one end of the liquid inlet tube is configured to introduce an external fluid, another end of the liquid inlet tube is connected to an inlet of the flow channel, and the flow channel and a space in the liquid inlet tube define at least a portion of the liquid inlet channel.

25. The endoscope according to claim 24, wherein the handle assembly comprises a handle, and a handgrip disposed at a bottom side of the handle, the working channel tube and the visual sleeve extend along a lengthwise direction of the handle, the mounting port is disposed at an end of the handle close to the insertion tube, at least a portion of the joint is disposed inside a distal end of the handle, and an end of the liquid inlet tube away from the joint extends along the handgrip to an end of the handgrip away from the handle.

26. The endoscope according to claim 23, wherein the endoscope fitting assembly comprises a turntable disposed outside the handle assembly, the turntable is arranged to cover around an outer peripheral side of the insertion tube, and the turntable is rotatably arranged to case around the joint and detachably fixed to the joint.

27. The endoscope according to claim 15, wherein the endoscope fitting assembly comprises an end cap, a proximal end surface of the handle assembly is provided with a first snapping part, the end cap is arranged to cover around the disc part, a circumferential surface of the end cap is provided with a second snapping part, and the first snapping part is detachably snapped to the second snapping part, to allow the disc part to be kept against a proximal end portion of the visual sleeve.

28. The endoscope according to claim 22, wherein the insertion tube assembly comprises a fixing support disposed inside a distal end of the insertion tube, the fixing support is connected to an inner wall of the insertion tube, the fixing support is provided with a first hole and a second hole, the working channel tube is constrained in the first hole, and the visual sleeve is constrained in the second hole, to allow a distal end of the working channel tube and a distal end of the visual sleeve to be fixed inside the insertion tube.

29. The endoscope according to claim 28, wherein a portion of an outer circumferential surface of the fixing support is recessed toward a direction away from the inner wall of the insertion tube to form a recessed region, and the recessed region and the inner wall of the insertion tube opposite to the recessed region define the liquid outlet.

30. The endoscope according to claim 28, wherein the fixing support comprises two mounting blocks separated from each other, the working channel tube and the visual sleeve are arranged along a first direction, and the two mounting blocks are disposed opposite to each other along a second direction and define the first hole and the second hole, wherein the first direction is perpendicular to the second direction.

31. The endoscope according to claim 30, wherein an outer circumferential surface of each mounting block facing toward the inner wall of the insertion tube is provided with an arc-shaped surface, and the arc-shaped surface is fitted against the inner wall of the insertion tube.

32. The endoscope according to claim 28, wherein the endoscope fitting assembly comprises a cable, and a light emitter configured to provide illumination to the image tube, the fixing support is provided with a through hole penetrating through the fixing support along an axial direction of the insertion tube, the cable passes through the through hole and is connected to the light emitter, and the inner wall of the insertion tube, an outer wall of the working channel tube, and an outer wall of the visual sleeve form a triangular region in which the cable is arranged.

33. The endoscope according to claim 32, wherein the light emitter comprises a circuit board, and a light emitting chip integrated on the circuit board, the circuit board is attached to a distal end surface of the fixing support, and a surface of the circuit board and a surface of the light emitting chip are coated with a glue filling layer.

34. The endoscope according to claim 28, wherein the insertion tube assembly comprises a glue filling layer, the glue filling layer is formed by filling glue into a region defined by a distal end surface of the fixing support, and the inner wall of the insertion tube and an outer wall of the optical window, and an outer surface of the glue filling layer forms at least a portion of the distal end surface of the insertion tube.

35. The endoscope according to claim 34, wherein the visual sleeve comprises a sheath and the optical window, a distal end of the sheath is open, the optical window closes a distal end portion of the sheath, and the glue filling layer is arranged between a circumference of the optical window and the inner wall of the insertion tube.

36. The endoscope according to claim 22, wherein the endoscope fitting assembly comprises a coupling part fixed to the handle assembly and a liquid discharging tube, the coupling part comprises a first coupling sleeve and a second coupling sleeve butted with each other, a distal end of the first coupling sleeve is provided with an insertion cavity, a proximal end of the second coupling sleeve is inserted into the insertion cavity, a cavity region is formed between a portion of a circumferential surface of the second coupling sleeve and an inner wall of the insertion cavity, a side wall of the insertion cavity corresponding to the cavity region is provided with a coupling interface, a proximal end of the working channel tube extends into and is fixed inside a distal end of the second coupling sleeve, and is in communication with the cavity region, a space expanding section that expands toward the first coupling sleeve is formed inside the proximal end of the second coupling sleeve, and an end of the liquid discharging tube is in communication with the coupling interface, to discharge fluid from the working channel tube.

37. The endoscope according to claim 36, wherein a cross-sectional area of the space expanding section increases gradually in a direction away from the working channel tube.

38. The endoscope according to claim 37, wherein the second coupling sleeve comprises a hermitic axial section, a circumferential surface of the hermitic axial section is in hermitic interference fit with the insertion cavity, an axial end surface of the hermitic axial section is spaced apart from a cavity end surface of the insertion cavity facing toward the axial end surface, and the coupling interface is disposed at a bottom of a circumferential side wall between the axial end surface and the cavity end surface.

39. The endoscope according to claim 38, wherein the second coupling sleeve comprises a dwindling axial section connected to a proximal end of the hermitic axial section, an outer diameter of the dwindling axial section is less than an outer diameter of the hermitic axial section, and the space expanding section extends from an interior of the hermitic axial section to a proximal end surface of the dwindling axial section.

40. The endoscope according to claim 39, wherein the first coupling sleeve is provided with a first axial hole, the insertion cavity is positioned at a distal end of the first axial hole, an inner diameter of the insertion cavity is greater than an inner diameter of the distal end of the first axial hole, the cavity end surface is formed at a boundary of the insertion cavity and the first axial hole, the first coupling sleeve is provided with an annular platform positioned in the insertion cavity, the annular platform surrounds a distal end portion of the first axial hole, and the annular platform is spaced apart from a circumferential side wall of the insertion cavity.

41. The endoscope according to claim 40, wherein an end surface of the annular platform is spaced apart from an end face of the dwindling axial section.

42. The endoscope according to claim 40, wherein a cross-sectional area of the first axial hole decreases continuously in a direction from a proximal end to the distal end of the first axial hole.

43. The endoscope according to claim 40, wherein the endoscope fitting assembly comprises a screw cap detachably arranged on a proximal end of the first coupling sleeve, the screw cap comprises a cap body and a bulge protruding from an interior of the cap body toward the first coupling sleeve, and the bulge extends into the first axial hole and seals the first axial hole.

44. The endoscope according to claim 36, wherein the handle assembly comprises a handle, and a handgrip disposed at a bottom side of the handle, the working channel tube and the visual sleeve extend along a lengthwise direction of the handle, and an end of the liquid discharging tube away from the coupling part extends along the handgrip to an end of the handgrip away from the handle.

45. The endoscope according to claim 44, wherein the endoscope fitting assembly comprises a coupling head, a first coupling cavity and a second coupling cavity in communication with each other are formed inside the coupling head, the first coupling cavity is in communication with the coupling interface, the second coupling cavity is in communication with an end of the liquid discharging tube close to the handle, the first coupling cavity is disposed along a lengthwise direction of the handgrip, and the second coupling cavity is disposed along the lengthwise direction of the handle.

46. The endoscope according to claim 45, wherein the liquid discharging tube comprises a first liquid discharging section, a bent section and a second liquid discharging section, a portion of the liquid discharging tube protrudes towards a direction away from the coupling part to form the bent section, the first liquid discharging section extends into and is fixed in the second coupling cavity, the second liquid discharging section extends along the lengthwise direction of the handgrip, and the bent section connects the first liquid discharging section and the second liquid discharging section.
